# EUROPEAN PATENT APPLICATION

(11) **EP 3 838 246 A1**
(43) Date of publication of application: **23.06.2021**
(21) Application number: 19850283.3
(22) Date of filing: 19.08.2019
(51) Int. Cl.: A61J 3/00, A61J 1/05

(54) **DRUG MANAGEMENT METHOD FOR KIT FORMULATION REQUIRING DOSE ADJUSTMENT**

(30) Priority: 17.08.2018 JP 2018153752
(71) Applicant: Regimen Kit, Inc., Tokyo 145-0072 (JP)
(72) Inventor: MIYAZAKI Futoshi, Kashima-shi Ibaraki 314-0025 (JP); ANDO Mamoru, Tokyo 145-0072 (JP)
(74) Representative: Zemanová, Veronika
(86) International application number: PCT/JP2019/032206
(87) International publication number: WO 2020/036231

(57) **Abstract**

The objective of the invention is to establish a novel kit formulation and a business model using this kit formulation, contributing to a mechanism for reducing the losses due to disposal of excess drugs, and to the suppression and the reduction of healthcare costs. The invention is the kit formulation combining a container housing a diluting or dissolving liquid such as pure water or an infusion, and a packaging container wherein the intended drug is contained, and a computer network-mediated managing method for the kit formulation.

## Description

### Technical Field

The present invention relates to the followings:
(1)A packaging container having a sensor (for example, temperature sensor, light sensor, humidity sensor, etc.) and identification mark that can record and update various information about drugs (for example, a recording medium such as an IC tag such as RFID and/or a two-dimensional barcode, a three-dimensional barcode, etc.),
(2) The drug container in which the desired drug is contained in the drug packaging container,
(3) A kit product in which a container containing a diluted/dissolved solution such as an infusion solution or pure water and a drug container containing the desired drug are combined,
(4) Management method of the kit product,
(5) Manufacturing method of the kit product, manufacturing apparatus thereof, and
(6) Recovery and/or reuse method of surplus drug using the management method of the kit product.

### Background Art

The drug to which the present technology is applied is characterized in that not only a fixed dose drug but also a drug that requires dose adjustment is targeted.
A drug that requires dose adjustment is a drug whose dosage varies depending on the individual patient, and is determined by the doctor based on one or more information selected from the following groups. is there.
1) Patient height
2) Weight
3) Age
4) Body surface area
5) Area under the blood concentration curve and serum creatinine value
6) Side effects of drug administration
7) Physical condition of patient

Drugs requiring dose adjustment include, for example, oral preparations, subcutaneous injection preparations, intramuscular injection preparations and intravenous injection preparations.
Among them, the injection is preferable, and the intravenous preparation is more preferable. The intravenous preparation includes a highly pharmacologically active drug and a non-highly pharmacologically active drug from the viewpoint of pharmacological activity, but the highly pharmacologically active drug is preferable.
Among the highly pharmacologically active drugs, anticancer agents are in great need.
In addition, the intravenous preparation includes high-risk drugs and Hazardous Drugs (HD) and non-high-dose drugs in terms of toxicity, but preferably there is a high need for high-risk drugs and Hazardous Drugs (HD).

Various kit products have been released for high calorie infusion formulations.
The kit product is a highly useful product because there is little risk of bacterial contamination and contamination with foreign substances, and the labor of preparation is eliminated.

Recently, NST (nutrition support team) activities have been expanded nationwide.
Optimal infusion composition for individual patients is considered in various occupations. (Chapter 3 Parenteral nutrition 2. Central parenteral nutrition (TPN) 17. TPN infusion preparation method (October 20, 2015 version) Non-patent document 1) And various kit products for high-calorie infusion formulations are described.
The kit product is a highly useful product because there is little risk of bacterial contamination and contamination with foreign substances, and the labor of preparation is eliminated.

By the way, hospital pharmacy departments and dispensing pharmacies, which perform dispensing operations in the medical field, handle an extremely large number of types of drugs. A problem at many medical institutions is the storage space for inventory and the complexity of management.
Sufficient amounts of medicinal products should be stored in the storage of each medical institution so that they can be used immediately when needed.

For example, OPDIVO (generic name: nivolumab) is sold as OPDIVO IV infusion 20 mg and OPDIVO IV infusion 100 mg.
As drug information regarding these drugs, regarding administration to patients requiring caution, the following measures are provided and recommended for handling when the administration solution is not immediately administered after preparation of the administration solution (Non-Patent Document 1). 2). "Be careful of bacterial contamination, so please administer as soon as possible.
If it is unavoidable and you cannot administer it immediately, store it in the refrigerator immediately.
Do not administer it since it has not been confirmed to be stable over 24 hours after dissolution and may cause bacterial contamination. "
Due to such circumstances, these drugs are not all used in therapy, and surplus drugs generated are basically reimbursed with full insurance and basically discarded.

On the other hand, expensive anti-cancer agents such as "OPDIVO" that act on immunity are appearing one after another.
Therefore, if the waste loss of the surplus drug that has not been used up is reduced, it is possible to expect medical cost reduction of several tens of billions of yen.
For this reason, the Ministry of Health, Labor and Welfare has established a policy of establishing safety standards so that the surplus anticancer agent can be effectively used for other patients.
The ministry plans to start a multifaceted research study on the safety of medical institutions and the effect of reducing medical costs when using surplus drug s (Ministry of Health, Labor and Welfare administrative contact on June 22, 2018 (2017 Health and Welfare Administration Promotion Survey Project Subsidy (Health and Labor Science Special Research Project) On "results for the creation of guidelines regarding") (Non-Patent Document 3)).

Examples of the prior art of the invention of the packaging container of the present invention include JP2009528866 (Patent Document 1) and JP2016062573 (Patent Document 2).

Patent Document 1 discloses several medical vial embodiments incorporating an RFID such that the RFID remains attached to the vial when the vial closure is opened.
In the preferred embodiment vial, the RFID is incorporated into the vial closure system.

Patent Document 2 discloses a packaging container having an elastic body with a built-in IC tag, in which the stress exerted on the IC tag when an external force acts is reduced and the durability is improved.
The deformation of the IC tag built-in elastic body 10' in which the IC tag 14 is arranged inside the elastic body 12 is allowed by the internal space 24'.

The following Patent Documents 3 to 7 are known as prior art of kit products.

JP2003220116 discloses a first compartment (A) containing a drug solution containing vitamin B2, a second compartment (B) containing a drug solution containing an iron source, and an infusion container containing the drug solution container including a communication mechanism capable of delivering to a container from both compartments. (Patent Document 3).

The following contents are described in JP2001524353.
- A container characterized in that the large compartments (10) contain bicarbonate ions and the small compartments (14, 15) contain glucose and calcium ions (US Pat. No. 6,037,049).
- This large compartment (10) is a container containing a medical solution, in particular for peritoneal dialysis, which is of sufficient volume to contain the finally prepared medicinal solution.
- These smaller compartments (14,15) are comprised of at least two smaller compartments containing a partial quantity of medical solution which is incompatible with the contents of the larger compartment during a long storage period.

JP2008539142 describes a liquid container (Patent Document 5) having the following features.
1) A programmable liquid container.
2) It has a main chamber that can be filled with the basic liquid.
3) It has a container inner surface in physical communication with the main chamber, a container outer surface, and a container with a sealable outlet.
4) A plurality of sealed additive chambers, each containing a separately selected additive and having a chamber with an inner surface and an outer surface.
5) The additive chamber outer surface is in physical communication with the container, and when the container is filled with a basic liquid and the outlet is sealed, the additive chamber can be opened by the user, the opening of the additive chamber allows the additive stored therein to communicate with the basic liquid in the container and does not compromise the integrity of the sealed container.

The following contents are described in JP2001299910.
One end of the cylinder is open.
Engaging means is provided at the open end.
An upper cylinder having a vent and an inlet at the other end, and one end of the cylinder opened.
Engaging means is provided at the open end.
A metering cylinder comprising a lower cylinder provided with an outlet on the other end. The outer diameter of one of the two types of cylinders is set to be slightly smaller than the inner diameter of the other cylinder.
One cylinder is inserted into the other cylinder, and the inner wall of one cylinder slides liquid-tightly on the outer wall of the other cylinder.
A fixed quantity tube characterized by changing the volume of the liquid medicine contained in the metering cylinder (Patent Document 6)

However, the following report discloses the state of the art of existing kit products for drugs that require dose adjustment.
1) Nakayama, Aki, Shiba, Saki, Yumiko, et al. "Advances in medical equipment for patients undergoing cancer chemotherapy (from the perspective of nutrition therapy)", Parenteral and enteral nutrition: Vol. 28, No. 2, 635(47)-644(56), 2013. (Non-patent document 4)
2) Round-table discussion TPN therapy considering medical safety Sponsor: Terumo Corporation (March 2013) (Non-patent document 5)

Specifically, Non-Patent Document 4 discloses the following contents.
- Regarding anticancer agents, it is difficult to formulate a kit because it often requires a detailed dosage design.
- In addition, there are some medicines where the use of in-line filters is restricted due to the specialization of the formulation.
- Furthermore, since not only sterility but also exposure countermeasures are important, there are currently no sufficient alternatives.
Further, Non-Patent Document 5 discloses the following contents.
- Certainly, if all necessary components are made into a prefilled syringe formulation, it will be unified and very easy to use, but unitization of the drug is contrary to individualization of medical care, so it is difficult to finely adjust the dose etc.
- This applies to all kit products, I think it is a very difficult issue to bridge the gap between improving efficiency and maintaining cleanliness and individualization.

Therefore, from the descriptions of Non-Patent Document 4 and Non-Patent Document 5, existing kit products have shown that individualized dose adjustments to patients are not possible.

The following contents are disclosed in WO2017038959 (Patent Document 7).
A kit product characterized by
a bag containing an infusion solution or pure water,
and at least one drug-enclosing portion in which a drug is enclosed and having a sealing part are provided,
and the drug-enclosing portion and the bag are connected via the sealing part, by opening the sealing part, the bag and the drug-enclosing portion communicate with each other, and a kit product in which the drug can be dispensed into the bag and can be prepared, and the dose of the drug can be adjusted.

JP4303900 discloses a business model for a drug replenishment method and its system, which is a business form in which a drug wholesaler grasps the customer's inventory status and delivers a drug (Patent Document 8).

### Citation List

### Patent Literature

Patent Document 1: JP2009-528866
Patent Document 2: JP2016-062573
Patent Document 3: JP2003-220116
Patent Document 4: JP2001-524353
Patent Document 5: JP2008-539142
Patent Document 6: JP 2001-299910 A
Patent Document 7: WO2017038959
Patent Document 8: JP4303900

### Non-Patent Literature

Non-Patent Document 1: Chapter 3 Parenteral Nutrition 2. Central Parenteral Nutrition (TPN) 17. TPN infusion preparation method (October 20, 2015 version)
Non-Patent Document 2: Opdivo Q&A (What should I do if I do not administer the dosing solution immediately after preparing it?)
Non-patent document 3: Administrative liaison with the Ministry of Health, Labor and Welfare on June 22, 2018 (FY2017 Health and Welfare Administration Promotion Survey Project Subsidy (Health and Labor Science Special Research Project) "Proper use of injectable anticancer agents, etc. "Research for the creation of guidelines for handling residual liquid" results
Non-Patent Document 4: Toshiaki Nakayama, Shiba ▲saki ▼Yumiko et al. "Advancement of medical equipment for patients undergoing cancer chemotherapy (from the viewpoint of nutrition therapy)", Parenteral and enteral nutrition: Vol. 28, No. 2, 635(47)-644(56), 2013.
Non-patent document 5: Round-table discussion TPN therapy considering from medical safety Sponsor: Terumo Corporation (March 2013)

### Summary of Invention

### Technical Problem

In medical practice, the number of drugs that require dose adjustment is increasing daily. There is a problem in that not only the drug but also a high-calorie infusion preparation, which requires a dose adjustment at the time of administration, is not sufficiently dealt with in the dispensing process.
In addition, the storage space is very narrow in many medical institutions.
In addition, drugs in inventory must be stored under appropriate temperature, humidity, and light conditions, and must be managed with a limited expiration date. It is necessary to manage and maintain inventory drugs in order to provide appropriate medical care, but there is a problem that they are not fully implemented.

In the case of an injection, the excess drug in the opened container must be stored aseptically, and it must be controlled whether the inside is kept sterile.
In addition, it is necessary to double- or triple-check whether the amount of surplus drug in the vial, which is a container for injection, is really the same as the indicated amount (whether there is a mistake in entry).
Therefore, these operations impose a heavy burden on medical worker who support the site with limited personnel.

The latest state of the art of existing kit products is specifically disclosed in Non-Patent Document 4 as follows.
- Regarding anticancer agents, it is difficult to formulate a kit because it often requires a detailed dose design.
- In addition, there are some medicines where the use of in-line filters is restricted due to the specialization of the formulation.
- Furthermore, since not only sterility but also exposure countermeasures are important, there are currently no sufficient alternatives.
Further, Non-Patent Document 5 has the following description.
- Certainly, if all necessary components are made into a prefilled syringe formulation, it will be unified and very easy to use, but unitization of the drug is contrary to individualization of medical care, so it is difficult to finely adjust the dose etc.
- This applies to all kit products , I think it is a very difficult issue to bridge the gap between improving efficiency and maintaining cleanliness and individualization.

Therefore, from the descriptions of Non-Patent Document 4 and Non-Patent Document 5, it has been shown that the existing kit product s cannot be individually dose-adjusted to patients. The creation of the kit product that enables dose adjustment to patients in any case is awaited.

The present inventor has disclosed, in Patent Document 7, a kit product having a basic configuration that enables prevention of exposure of a pharmacist by, for example, an anticancer agent and dose adjustment of the drug to a patient.

However, the kit product has not been designed in consideration of the dispensing act in the medical practice and the use in the treatment of patients, and a new kit product that meets the use is required.

Furthermore, as in Non-Patent Document 3, a mechanism for reducing the waste loss of the surplus drug has just begun to be examined.
The establishment of such a system and the control and reduction of medical expenses are major issues.
Furthermore, a new kit product that contributes to the solution of the problem is required.

The existing drug management system comprises a demand forecasting unit, a drug replenishment mode selecting unit, and a delivery plan creating unit in order to reduce the number of emergency deliveries.
Therefore, there is a demand for a new business model that contributes to the support of dispensing activities in the medical practice and reduction of waste loss of surplus drugs.

### Solution to Problem

As a result of various studies to solve the above problems to be solved, the present inventors have found a new method for recovering and/or reusing a surplus drug having the following elements, and completed the present invention.
- Drug packaging container having recording medium and/or identification mark
- Containers for drug packaging containing drugs
- Kit products
- Management method of kit products
- Method for manufacturing kit products
- Computer program for Management method of kit products

[1] A packaging container for a drug having a recording medium and/or an identification mark for recording and updating one or more arbitrary information(s) selected from the group consisting of the information (1) to (9):
   (1) An active ingredient of a drug
   (2) Usage of a drug
   (3) Drug dose
   (4) Date of manufacture of a drug
   (5) Expiration date of a drug
   (6) Drug shipping date of a drug
   (7) Traceability of drug delivery route from manufacturing to usage of a drug, and storage conditions (eg. temperature, light (dark/light), humidity, etc.) including storage temperature and changes thereof
   (8) Information including the administration date of a drug and a breakdown of the administration
   (9) Amount of drug presented in drug-enclosing portion
[2] The drug container according to [1], further containing the drug which corresponds to (1) to (9) information about the drug concerned.
[3] The drug container according to [2], wherein the drug dose is fixed or determined based on patient information of the usage and dose for the patient.
[4] The drug container according to [2] or [3], wherein the drug for a patient is an injection for a disease selected from the group consisting of 1) to 17) below.
   Group:
   1) Sensory medicine:
      (a) Verteporfin
      (b) Aflibercept, diphenhydramine hydrochloride/diprophylline, triamcinolone acetonide, pegaptanib sodium, ranibizumab
   2) Central nervous system drugs:
      (a) Acetaminophen, ketamine hydrochloride, droperidol, phenobarbital sodium, buprenorphine hydrochloride, propofol, phosphenytoin sodium hydrate, midazolam, levetiracetam
      (b) Apomorphine hydrochloride hydrate, aripiprazole hydrate, edaravone, eptazocine hydrobromide, olanzapine, clomipramine hydrochloride, chlorpromazine hydrochloride, ketoprofen, chondroitin sulfate sodium sodium salicylate, sodium salicylate, sodium salicylate/dibucaine combination drug, diazepam, diclofenac sodium, sulpiride, sulpirine hydrate, secobarbital sodium, timiperone, dexmedetomidine hydrochloride, tramadol hydrochloride, natalizumab, nucinercene sodium, paliperidone palmitate, paroxetine hydrochloride hydrate, haloperidol, Haloperidol decanoate, hydroxyzine hydrochloride, phenytoin sodium, phenobarbital, flunitrazepam, fluphenazine decanoate, flurbiprofen axetil, prochlorperazine mesylate, pentazocine, methamphetamine hydrochloride, lacosamide, risperidone, levodopa, levomepromazine hydrochloride, lorazepam, vaccinia virus inoculated rabbit inflamed skin extract, perphenazine hydrochloride, thiamiral sodium for injection, thiopental sodium for injection, biperidene lactate, chloral hydrate
   3) Peripheral nerve drugs
      (a) suxamethonium chloride hydrate, dantrolene sodium hydrate, suxamethonium chloride hydrate for injection, vecuronium bromide, lidocaine, rocuronium bromide
      (b) type A botulinum toxin, type B botulinum toxin, atropin sulfate hydrate, scopolamine hydrobromide hydrate, thymepidium bromide hydrate, tetracaine hydrochloride, neostigmine methyl sulfate, neostigmine methyl sulfate / atropin sulfate hydrate, bacrophen, papaverin hydrochloride, butylscopolamine bromide, bupivacaine hydrochloride hydrate, prizinol mesylate, procaine hydrochloride, mepivacaine hydrochloride, lidocaine hydrochloride, lidocaine hydrochloride / adrenaline, ropivakine hydrochloride hydrate, levobpivacaine hydrochloride, acetylcholine chloride for injection, magnesium sulfate hydrate, magnesium sulfate hydrate / glucose
   4) Cardiovascular medicine
      (a) aplindine hydrochloride, aminophylline hydrate, ibuprofen L-lysine, indomethacin sodium, esmolol hydrochloride, cibenzoline succinate, digoxin, deslanoside, dopamine hydrochloride, nifekalant hydrochloride, pilsicainide hydrochloride hydrate, flecainide acetate, bucladesine sodium, verapamil hydrochloride, milrinone anhydrous caffeine
      (b) D-mannitol, 1-isoprenaline hydrochloride, sodium acetazolamide, amiodarone hydrochloride, allilocoumab, argatroban hydrate, alprostadil, alprostadil alfadex, isoxuprine hydrochloride, etilefrine hydrochloride, epoprostenol sodium, epoprostenol sodium solution, evolocumab, olprinone hydrochloride hydrate, carperitide, candesartan cilexetil, potassium canrenoate, colforsin daropate hydrochloride, citicoline, dipyridamole, diprophylline, diltiazem hydrochloride, sumatriptan succinate, dobutamine hydrochloride Salt, Treprostinil, nicardipine hydrochloride, nicorandil, nitroglycerin, sodium nitroprusside hydrate, fasudil hydrochloride hydrate, phenylephrine hydrochloride, bumetanide, procainamide hydrochloride, proxyfilin, furosemide, anhydrous caffeine, propranolol hydrochloride, mexiletine Hydrochloride, landiolol hydrochloride, lidocaine, isosorbide nitrate, hydralazine hydrochloride for injection, concentrated glycerin/fructose, anhydrous caffeine
   5) Respiratory medicine
      (a) Doxapram hydrochloride hydrate
      (b) dl-methylephedrine hydrochloride, acetylcysteine, ephedrine hydrochloride, omalizumab, guaifenesin, dimorphoramine, dextromethorphan hydrobromide hydrate, terbutaline sulfate, naloxone hydrochloride, flumazenil, bromhexine hydrochloride, venralizumab, mepolizumab, levallorphan tartrate, lung surfactant
   6) Drugs for digestive organs
      (a) infliximab (genetical recombination), granisetron hydrochloride, fosaprepitantomeglumine,, ondansetron hydrochloride hydrate
      (b) azasetron hydrochloride, omeprazole sodium, carnitine chloride, cimetidine, sulpiride, dehydrocholic acid, palonosetron hydrochloride, famotidine, budesonide, vedolizumab (genetical recombination), berberine sulfate hydrate, mesalazine, ranitidine hydrochloride, ramosetron Hydrochloride, lansoprazole, roxatidine acetate hydrochloride, metoclopramide hydrochloride, calf blood extract
   7) Hormonal agents (including antihormonal agents)
      (a) Somatropin (genetical recombination), prednisolone sodium succinate for injection, desmopressin acetate hydrate, hydrocortisone sodium succinate, mechasermine (genetical recombination), methylprednisolone sodium succinate
      (b) adrenaline, insulin aspart (genetical recombination), insulin glargine (genetical recombination), insulin glulisine (genetical recombination), insulin degludec (genetical recombination), insulin degludec (genetical recombination) insulin aspart (gene combination) combination, insulin detemir (genetical recombination), insulin human (genetical recombination), insulin lispro (genetical recombination), exenatide, estradiol valerate, estriol, oxytocin, octreotide acetate, ganirelix acetate, guest noroncaproate, gonadorelin acetate, coriogonadotropin alpha (genetical recombination), dinoprost, cetrorelix acetate, semaglutide (genetical recombination), thiamazole, degarelix acetate, dexamethasone palmitate, dexamethasone phosphate sodium, testosterone enanthateacid ester, testosterone enanthate/estradiol valerate, tetracosactide acetate, dulaglutide (genetical recombination), teriparatide (genetical recombination), teriparatide acetate, triamcinolone acetonide, noradrenaline, pasireotide pamoate, vasopressin, hydroxyprogesterone capron acid ester, hydroxyprogesterone caproate ester/estradiol benzoate ester, hydrocortisone sodium phosphate ester, follitropin alfa (genetical recombination), buserelin acetate salt, plasterone sulfate sodium hydrate, prednisolone sodium phosphate salt, progesterone, pegvisomant (genetical recombination), betamethasone phosphate sodium, betamethasone acetate/betamethasone phosphate sodium, methylprednisolone acetate, metenolone enanthate, metreleptin (genetical recombination), lanreotide acetate, lixisenatide, leuprorelin Acetate, liraglutide (genetical recombination), pituitary gonadotropin, human chorionic gonadotropin for injection
   8) Drugs for blood and body fluids
      (a) dalteparin sodium, dextran 40/glucose, thrombomodulin alpha (genetical recombination), naltograstim (genetical recombination), parnaparin sodium, hydroxyethyl starch 70000, prelixafor, polidocanol, lenograstim (genetical recombination), filgrastim (genetical recombination)
      (b) 10% sodium chloride, idalcizumab, enoxaparin sodium, sodium carbazochrome sulfonate hydrate, danapaloid sodium, tranexamic acid, trometamol, fondaparinux sodium, protamine sulfate, pegfilgrastim, heparin calcium, heparin sodium, hemocoagulase, mirimostim, monoethanolamine oleate, ringer's solution, dipotassium phosphate, reviparin sodium, maintenance solution, maintenance solution (with xylitol), maintenance solution (with glucose), maintenance solution (with complex sugar), potassium chloride, sodium chloride, starting solution, hot water extract of Mycobacterium tuberculosis, bicarbonate Ringer's solution, postoperative recovery solution, acetate ringer's solution, acetate Ringer's solution (glucose added), acetic acid maintenance solution, acetic acid maintenance solution (glucose added), physiological saline solution, dehydration Replenisher, Ringer's lactate, Ringer's lactate (with sorbitol), Ringer's lactate (with Dextran 40), Ringer's lactate (with glucose), Ringer's lactate (with maltose)
   9) Other metabolic drugs
      (a) abatacept (genetical recombination), alglucosidase alpha (genetical recombination), alteplase (genetical recombination), imiglucerase (genetical recombination), erosulfase alpha (genetical recombination), gabexate mesylate, cyclosporine, cibelestat sodium hydrate, sugammadex sodium, sebelipase alpha (genetical recombination), tacrolimus hydrate, sodium bicarbonate, darbepoetin alfa (genetical recombination), deferoxamine mesylate, hydroxocobalamin berimumab (genetical recombination), fomepizole, methylthioninium chloride hydrate, monteplase (genetical recombination), rasburicase (genetical recombination), lalonidase (genetical recombination), dexrazoxane, levofolinate calcium
      (b) L-arginine L-glutamate hydrate, L-arginine hydrochloride, agarcidase alpha, agarcidase beta, asphotase alpha, adalimumab, adenosine triphosphate disodium hydrate, alendronate sodium hydrate, ixexumab, idulsulfase, ibandronate sodium hydrate, ustekinumab, ulinastatin, urokinase, etanercept, calcium disodium edetate hydrate, etelcalcetide hydrochloride, epoetin alfa, epoetin kappa, epoetin beta pegol, epoetin beta, elapegade mase, elcatonin, ozagrel sodium, canakinumab, calcitonin (salmon) , gallsulfase, gusperimus hydrochloride, guselcumab, glatiramer acetate, glycyrrhizin/glycine/cysteine combination drug, glutathione, golimumab, chondriase, chondroitin sulfate sodium salt, salilumab, dimerkaprol, secukinumab, certolizumab pegol, zoledronic acid hydrate, thioctic acid, sodium thiosulfate hydrate, denosumab, nafamostat mesylate, batroxobin, disodium pamidronate hydrate, Sodium hyaluronate cross-linked polymer / sodium hyaluronate cross-linked polymer Vinyl sulfone cross-linked product, sodium hyaluronate cross-linked polymer hyaluronic acid sodium cross-linked polymer vinyl sulfone cross-linked product pralidoxime iodide, brodalumab, iron(III) hexacyanoferrate (II) hydrate, veraglucerase alpha, calcium tripentate trisodium, zinc tripentate trisodium, holinate calcium, mesna, rsankizumab, levocarnitine, lomiprostim, romosozumab, purified sodium hyaluronate
   10) Drugs for tumors
      (a) Actinomycin D, aclarubicin hydrochloride, aflibercept beta (genetical recombination) , avelumab (genetical recombination) , yttrium (90Y) ibritumomab tiuxetane (genetical recombination) , ipilimumab (genetical recombination) , ifosfamide, elotuzumab (Genetical recombination) , radium chloride (223Ra) , carboplatin, cladribine, arsenic trioxide, cyclophosphamide hydrate, cytarabine, daratumumab (genetical recombination) , vinblastine sulfate for injection, durvalumab (genetical recombination) , trastuzumab (genetical recombination), trastuzumab emtansine (genetical recombination), doxorubicin hydrochloride, nivolumab (genetical recombination) , nimustine hydrochloride, panitumumab (genetical recombination) , vincristine sulfate, vindesine sulfate, fluorouracil, busulfan, brentuximab vedotin (genetical recombination) , bevacizumab (genetical recombination) , pembrolizumab (genetical recombination) , porfimer sodium, methotrexate, mogamulizumab (genetical recombination) , ramucirumab (genetical recombination), azacitidine, amrubicin hydrochloride, idarubicin Hydrochloride, Yttrium (90Y) ibritumomab thiuxetan (genetical recombination) , inotuzumab ozogamicin (genetical recombination) , ifosfamide, irinotecan hydrochloride hydrate, etoposide, epirubicin hydrochloride, eribulin mesylate, oxaliplatin, cabazitaxel acetone adduct, carfilzomib, carboplatin, gemcitabine hydrochloride, gemtuzumab ozogamicin (genetical recombination) , cyclophosphamide hydrate, cisplatin, cytarabine, streptozocin, cetuximab (genetical recombination) , talaporfin sodium, dacarbazine for injection, vinblastine sulphate, temozolomide, trabectedine, doxorubicin hydrochloride, docetaxel, nedaplatin, nelarabine, nogitecan hydrochloride, paclitaxel, vinorelbine tartrate, vincristine sulphate, pirarubicin hydrochloride, fluorouracil, fludarabine phosphate, bleoromycin hydrochloride, pralatrexate, bendamustine hydrochloride, pemetrexed sodium hydrate, pentostatin, bortezomib, mitoxantrone hydrochloride, methotrexate, melphalan, ranimustine, rituximab (genetical recombination) , romidepsin
      (b) L-asparaginase, atezolizumab, alemtuzumab, enocitabine, obinutuzumab, ofatumumab, clofarabine, daunorubicin hydrochloride, thiotepa, tegafur, temsirolimus, paclitaxel (albumin suspension type) , brinatumomab, fulvestrant, pepromycin sulphate, pertuzumab, mitomycin C, miriplatin hydrate, pemetrexed sodium hydrate, pertuzumab (genetical recombination) , bendamustine hydrochloride, pentostatin, bortezomib, porfimer sodium, mitomycin C, mitoxantrone hydrochloride, miriplatin hydrate, lentinan, anhydrous Ethanol, sterilized talc, Streptococcus faecalis extract
         (Alkaloid narcotics) Opium alkaloid atropine, opium alkaloid scopolamine, opium alkaloid hydrochloride, oxycodone hydrochloride hydrate, hydromorphone hydrochloride, morphine atropine, morphine hydrochloride hydrate, weak opium alkaloid scopolamine, compound oxycodone, compound oxycodone/atropine
   11) Radiopharmaceutical
      (a) Strontium chloride (89Sr), gallium citrate (67Ga)
      (b) N-pyridoxyl-5-methyltryptophan technetium (99mTc) , ioflupane (123I) , iomazenil (123I) , indium (111In) ibritumomab thixetane, indium (111In) pentetreotide, examethazim technetium (99mTc) , ethylenedisisty nate oxotechnetium (99mTc) , ethylenebiscysteine diethyl ester dihydrochloride, galactosyl human serum albumin technetium diethylenetriamine pentaacetate (99mTc) , krypton (81mKr) , indium diethylenetriaminepentaacetate (111In) , dimercaptosuccinic acid, dimercaptosuccinic acid technetium (99mTc) , tin colloid, technetium large aggregate human serum albumin (99mTc), tetrofosmin technetium (99mTc) , hydroxymethylenediphosphonic acid, technetium hydroxymethylenediphosphonate (99mTc) , fludeoxyglucose (18F), flutetamol (18F), florbetapyr (18F), hexakismethoxyisobutylisonitrile technetium (99mTc), metaiodobenzylguanidine (123I), technetium methylenediphosphonate (99mTc), mercaptoacetylglycylglycylglycine technetium (99mTc) , methyl iodide norcholestenol (131I), iodophenylmethylpentadecanoic acid (123I), indium chloride (111In), thallium chloride (201Tl), N-Isopropyl-4-iodoamphetamine hydrochloride (123I), sodium pertechnetate (99mTc) , peroxide Sodium technetate (99mTc) generator, human serum albumin diethylenetriamine technetium pentaacetate (99mTc)
   12) Antibiotic preparation
      (a) aztreonam, amikacin sulfate, amphotericin B, arbekacin sulfate, ampicillin sodium/cloxacillin sodium hydrate, ampicillin sodium/sulbactam sodium, ampicillin sodium, imipenem hydrate/cilastatin sodium, quinupristin/dalfopristin, culindamycin phosphate, gentamicin sulfate, colistin methanesulfonate sodium, cefazolin sodium, cephalothin sodium, cefozopran hydrochloride, cefotaxime sodium, cefotiam hydrochloride, cefoperazone sodium sulbactam sodium, ceftazidime hydrate, ceftriaxone sodium water Japanese syrup, cefpirom sulfate, cefminox sodium hydrate, cefmetazole sodium, cefmenoxime hydrochloride, tazobactam sodium piperacillin sodium, daptomycin, teicoplanin, tobramycin, doripenem hydrate, vancomycin hydrochloride, panipenem betamipron, piperacillin sodium, flomoxef sodium, fosfomycin sodium, voriconazole, micafungin sodium, meropenem hydrate, latamoxef sodium-Mg/m2: caspofungin acetate
      (b) azithromycin hydrate, isepamycin sulfate, erythromycin lactobionate, enviomycin sulfate, kanamycin sulfate, chloramphenicol sodium succinate, dibekacin sulfate, streptomycin sulfate, spectinomycin hydrochloride hydrate, cefepime hydrochloride hydrate, ceftrozan sulfate/tazobactam sodium, tigecycline, biapenem, benzylpenicillin potassium, minocycline hydrochloride, lincomycin hydrochloride hydrate
   13) Chemotherapeutic agents
      (a) isoniazid, ciprofloxacin, vidarabine, fluconazole, peramivir hydrate, foscarnet sodium hydrate, linezolid
      (b) acyclovir, itraconazole, ganciclovir, tedizolidolinic acid ester, pazufloxacin mesylate, palivizumab, phosfluconazole, miconazole, letermovir, levofloxacin hydrate
   14) Biological products
      (a) albutrepenonacog alpha (genetical recombination) , antithrombin gamma (genetical recombination) , eptacog alfa (active form) (genetical recombination) , dried ion-exchange resin treated human immunoglobulin, dried sulfonated human immunoglobulin, dried concentrated human antithrombin III, dried concentrated human activated protein C, dried pH 4 treated human immunoglobulin, dried polyethylene glycol treated human immunoglobulin, antihuman thymocyte rabbit immunoglobulin, turoctocog alfa (genetical recombination) , tocilizumab (gene Recombination) , nonacog alpha (genetical recombination), nonacog gamma (genetical recombination) , nonacog beta pegol (genetical recombination) , hemin bezlotoxumab (genetical recombination) , polyethylene glycol treated anti-HBs human immunoglobulin, polyethylene glycol treated human immunoglobulin, lurioctocog alfa pegol (genetical recombination) , lonoctocog alfa (genetical recombination) , interferon gamma-la (genetical recombination)
      (b) tetravalent meningococcal vaccine, pH4-treated acidic human immunoglobulin, interferon alpha (NAMALWA) , interferon alpha-2b, interferon beta, interferon beta-la, interferon beta-1b, eculizumab, eftrenonacog alfa, ephraloctocog alfa, emicizumab, octocog beta, octocog alfa, catridecacog, sermoleukin, damoctocog alfa pegol, tesseleukin, basiliximab, histamine-complemented immunoglobulin (dry), human plasma-derived dry blood coagulation factor XIII, fibrinogen-containing factor XIII, fibrinogen combination agent, peginterferon alpha-2a, peginterferon alpha-2b, polyethylene glycol-treated anti-tetanus human immunoglobulin, lurioctocog alfa (genetical recombination) , heated human plasma protein, dried BCG for intravesical use (Japanese strain), dried gas esophagus horse Antitoxin, dried diphtheria horse antitoxin, dried habuma antitoxin, dried pepsin-treated human immunoglobulin, dried botulinum antitoxin, dried mamushi horse antitoxin, dried anti-D(Rho) human immunoglobulin, dried anti-HBs human immunoglobulin, dried anti- tetanus human immunoglobulin, dried cell culture smallpox vaccine, dried cell culture Japanese encephalitis vaccine, dried attenuated mumps vaccine, dried attenuated rubella vaccine, dried attenuated measles vaccine, dried attenuated measles and rubella mixed vaccine, dried human fibrinogen, dried Human blood coagulation factor antibody bypass activity complex, dried human blood coagulation factor IX complex, dried recombinant herpes zoster vaccine, dried tissue culture inactivated hepatitis A vaccine, dried tissue culture inactivated rabies vaccine, dried concentrated human C1- Inactivator, dried concentrated human prothrombin complex, dried concentrated human blood coagulation factor IX, dried concentrated human blood coagulation factor VIII, dried concentrated human blood coagulation factor X activated factor VII, anti-HBs human immunoglobulin, anti-tetanus human immunoglobulin, human haptoglobin, human serum albumin, human serum albumin (genetical recombination) , human immunoglobulin, purified tuberculin, recombinant precipitated tetravalent human papillomavirus-like particle vaccine, recombinant precipitated hepatitis B vaccine, precipitated 13 -Valent Streptococcus pneumoniae-binding vaccine, precipitated purified Pertussis diphtheria tetanus inactivated polio (Sabin strain) mixed vaccine, precipitated purified Pertussis diphtheria tetanus inactivated polio (soak vaccine) Combination vaccine, inactivated polio vaccine,
         (Antibody) adalimumab, atezorizumabu, aberumabu, arirokumabu, alemtuzumab, ikisekizumabu, idarucizumab, yttrium (90Y) ibritumomab tiuxetan, inotsuzumabu ozogamicin, ipilimumab, indium (¹¹¹In) ibritumomab tiuxetan, infliximab, usutekinumab, eculizumab, evolocumab, emishizumab, erotsuzumab, obinutsuzumab, ofatumumab, omalizumab, canakinumab, guselkumab, gemtuzumab ozogamicin, golimumab, salilumab, secukinumab, cetuximab, certolizumab pegol, daratumumab, denosumab, dupilumab, durbalumab, tosirimab, trastuzumab emtansin, trastuzumab, natalizumab, nivolumab, pasiliximab, panitumumab, palivizumab, busulfan, blinatumomab, brentuximab vedotin, brodalumab, vezlotoxumab, vedolizumab, bevacizumab, pembrolizumab, belimumab, pertuzumab, benlalizumab,mepolizumab, mogamulizumab, ranibizumab, ramucirumab, risankizumab, rituximab, romosozumab
   15) Parasite medicine
      (a) sulfamethoxazole/trimethoprim, pentamidine isethionate
      (b) metronidazole
   16) Diagnostic drugs (excluding in vitro diagnostic drugs)
      (a) iohexol, iomeprol, indocyanine green, gadoxetate sodium, gadodiamide hydrate, gadoteridol, gadoteric acid meglumine, gadobutolol, gadopentetate meglumine, sodium paraaminohippurate, phenolsulfonphthalein, felcarbotran, perflubutane, teriparatide acetic acid salt
      (b) L-arginine hydrochloride, sodium amidotrizoate meglumine, ioxaglic acid, iodixanol, meglumine iothroxate, iotrolan, iopamidol, iopromide, ioversol, inulin, indigo carmine, edrophonium chloride, galactose-palmitic acid mixture, glucagon, glucagon ( (Genetical recombination) , gonadorelin acetate, corticorelin (human) , somatrelin acetate, phentolamine mesylate, pralmorelin hydrochloride, fluorescein, protilerin, protylerin tartrate hydrate, iodinated poppy oil fatty acid ethyl ester, barium sulfate
   17) Non-alkaloid narcotics
      (a) droperidol/fentanyl citrate, fentanyl citrate, remifentanil hydrochloride
      (b) pethidine hydrochloride, pethidine hydrochloride/levallorphan tartrate
[5] A series of drug containers according to [2], each of which contains at least one drug dose selected from the group consisting of 1x, 2x, 3x, 4x, 5x, 6x, 7x, 8x, 9x, 10x, 20x, 30x, 40x, 50x, 60x, 70x, 80x, 90x and 100x (wherein x indicates a multiplier of 0.001, 0.01, 0.1, 1.0, 10, 100, and 1000 depending on the approved prescribed dose of a drug (unit: µg, mg, g) to be used for dose adjustment of the administration drug.
[6] A kit product comprising a container containing a diluted or dissolved solution including an infusion solution or pure water and a plurality of drug enclosing portions having sealing part (wherein the said drug is enclosed in the packaging container according to [1], and the container and the drug enclosing portions are brought into communication with each other by connecting the drug container in which the drug is enclosed to the packaging container via the sealing part and opening the sealing part) and the series of drug containers having the drug according to [5].
[7] The kit product according to [6], wherein the drug can be prepared by being placed in a container containing a diluted or dissolved solution, and the dose of the drug is selected based on patient information, by selecting the drug and the drug enclosing portions to adjust the dose of the drug and the dosage of the drug based on significant digit of the dose characterized in a constitution of a drug to be administered and a drug to be separated and not administered.
[8] The kit product according to [6] or [7], which comprises a container containing a diluted or dissolved solution including an infusion solution or pure water, plural drug enclosing portions having sealing part(s), and plural drug containers selected from a series of drug containers.
[9] A method for managing a kit product containing a selected drug container and a non-selected drug container characterized in comprising the following steps (1) to (7):
   (1) A step of providing medication prescription
   (2) A step of providing the kit product according to [6] based on the medication prescription
   (3) A step of providing a drug container selected for dispensing of the kit product
   (4) A step of drug management including a step of providing a non-selected drug container for dispensing the kit product
   (5) Classification of selected drug containers and non-selected drug containers in the above kit product
   (6) Providing data on the selected drug containers and information thereof based on the above classification results
   (7) Providing data on non-selected drug containers and information thereof based on the above classification results.
[10] A control method of computer wherein the computer system includes a data storage area which is connected to the terminals of the medical provider, the dispensing provider, and the pharmaceutical provider and a data storage unit where the drug manufacturer and/or drug supplier store the addresses of the terminals thereof, and the data storage area is executed to manage a selected drug container and the drug thereof and a non-selected drug container and the drug thereof characterized in including the following steps (1) to (8) via computer network:
   (1) A step of receiving data of medication prescription from the terminal of the medical provider
   (2) A step of receiving the data of the kit product according to [6] from the terminals of the drug manufacturer and/or the drug supplier based on the data of the medication prescription
   (3) A step of receiving the data of a selected drug container for dispensing of the kit product at terminals of a dispensing provider, a pharmaceutical provider, and a drug manufacturer and/or a drug supplier
   (4) A step of controlling a computer including a step of receiving the data of a non-selected drug container for dispensing of the kit product at terminals of the dispensing provider, the pharmaceutical provider, and the drug manufacturer and/or the drug supplier
   (5) Classification of selected drug containers and non-selected drug containers in the above kit product
   (6) Accepting the data of the selected drug container and information thereof based on the above classification result at the terminals of the dispensing provider, the pharmaceutical provider, and the drug manufacturer and/or drug supplier
   (7) Separation and collection of the non-selected drug containers on the basis of the above classification result, in response to receiving the data regarding the non-selected drug containers and information thereof at the terminals of the dispensing provider, the pharmaceutical provider, and the drug manufacturer and/or drug supplier
   (8) The step of transmitting the processing information regarding the separation and the collection to the terminals of the medical provider, the dispensing provider, and the pharmaceutical provider.
[11] A computer system wherein the computer system includes a data storage area which is connected to the terminals of the medical provider, the dispensing provider and a data storage unit where the drug manufacturer and/or drug supplier store the addresses of the terminals thereof, and the data storage area carries out management of a selected drug container and the drug thereof and a non-selected drug container and the drug thereof characterized in including the following steps (1) to (8) via computer network:
   (1) A step of receiving data of medication prescription from the terminal of the medical provider
   (2) A step of receiving the data of the kit product according to [6] from the terminals of the drug manufacturer and/or the drug supplier based on the data of the medication prescription
   (3) A step of receiving the data of a selected drug container for dispensing of the kit product at terminals of a dispensing provider, a pharmaceutical provider, and a drug manufacturer and/or a drug supplier
   (4) A step of controlling a computer including a step of receiving the data of a non-selected drug container for dispensing of the kit product at terminals of the dispensing provider, the pharmaceutical provider, and the drug manufacturer and/or the drug supplier
   (5) Classification of selected drug containers and non-selected drug containers in the above kit product
   (6) Accepting the data of the selected drug container and information thereof based on the above classification result at the terminals of the dispensing provider, the pharmaceutical provider, and the drug manufacturer and/or drug supplier
   (7) Separation and collection of the non-selected drug containers on the basis of the above classification result, in response to receiving the data regarding the non-selected drug containers and information thereof at the terminals of the dispensing provider, the pharmaceutical provider, and the drug manufacturer and/or the drug supplier
   (8) Transmission of the processing information regarding the separation and the collection to the terminals of the medical provider, the dispensing provider, and the pharmaceutical provider.
[12] A computer program wherein the computer system includes a data storage area which is connected to the terminals of the medical provider, the dispensing provider, and the pharmaceutical provider and a data storage unit where the drug manufacturer and/or drug supplier store the addresses of the terminals thereof, and the data storage area carries out management of a selected drug container and the drug thereof and a non-selected drug container and the drug thereof characterized in performing the following steps (1) to (8) via computer network:
   (1) A step of receiving data of medication prescription from the terminal of the medical provider
   (2) A step of receiving the data of the kit product according to [6] from the terminals of the drug manufacturer and/or the drug supplier based on the data of the medication prescription
   (3) A step of receiving the data of a selected drug container for dispensing of the kit product at terminals of a dispensing provider, a pharmaceutical provider, and a drug manufacturer and/or a drug supplier
   (4) A step of controlling a computer including a step of receiving the data of a non-selected drug container for dispensing of the kit product at terminals of the dispensing provider, the pharmaceutical provider, and the drug manufacturer and/or the drug supplier
   (5) Classification of selected drug containers and non-selected drug containers in the above kit product
   (6) Accepting the data of the selected drug container and information thereof based on the above classification result at the terminals of the dispensing provider, the pharmaceutical provider, and the drug manufacturer and/or drug supplier
   (7) Separation and collection of the non-selected drug containers on the basis of the above classification result, in response to receiving the data regarding the non-selected drug containers and information thereof at the terminals of the dispensing provider, the pharmaceutical provider, and the drug manufacturer and/or the drug supplier based on the classification result
   (8) Transmission of the processing information regarding the separation and the collection to the terminals of the medical provider, the dispensing provider, and the pharmaceutical provider.
[13] A method for producing a kit product characterized in comprising preparation of a series of drug containers according to [2], and combination of plural drug-enclosing portions having a container containing the diluted or dissolved solution including an infusion solution or pure water according to [6] and a sealing part, and the series of drug containers by using the method for managing a kit product according to [9] to [11].
[14] A process for producing a kit product characterized in comprising preparation of a series of drug containers according to [2], and combination plural drug-enclosing portions having a container containing the diluted or dissolved solution including an infusion solution or pure water according to [6] and a sealing part, and the series of drug containers by using the method for managing a kit product according to [9] to [11].
[15] The method for producing a kit product according to [13] characterized in further using the recovered non-selected drug container according to (4) of [9].
[16] A method for separating, recovering and/or reusing a non-selected drug container, characterized in combining the method for managing a kit product according to [9] and the method for preparation of a kit product according to [13].
[17] A control method of computer for administration of the kit product according to [10] wherein the computer system includes a data storage area which is connected to each terminal of medical workers and a data storage unit where the medical workers store each the addresses of the terminals thereof, the data storage area is characterized in including the following means (1) to (3) via computer network:
   (1) Means for a doctor to transmit a medication prescription relating to the kit product according to [6], and to transmit and receive the data of the medication prescription among the terminals of medical workers other than the doctor
   (2)Means for transmitting or receiving the data regarding the selected drug containers and the information thereof and the data regarding the non-selected drug containers and the information thereof, and/or the data on a container containing a diluted or dissolved solution including an infusion solution or pure water and plural drug-enclosing portions having sealing part(s) among each terminal of medical workers in case of the kit product according to [6] based on the data of the medication prescription, classifying selected drug containers and non-selected drug containers is settled
   (3) Means for transmitting and receiving processing information regarding separation and collection of the non-selected drug container among each terminal of medical workers.
[18] A computer system for administration of the kit product according to [10], which includes a data storage area which is connected to each terminal of medical workers and a data storage unit where the medical workers store each the addresses of the terminals thereof, the data storage area is characterized in including the following means (1) to (3) via computer network:
   (1) Means for a doctor to transmit a medication prescription relating to the kit product according to [6], and to transmit and receive the data of the medication prescription among the terminals of medical workers other than the doctor
   (2) Means for transmitting or receiving the data regarding the selected drug containers and the information thereof and the data regarding the non-selected drug containers and the information thereof, and/or the data on a container containing a diluted or dissolved solution including an infusion solution or pure water and plural drug-enclosing portions having sealing part(s) among each terminal of medical workers in case of the kit product according to [6] based on the data of the medication prescription, classifying selected drug containers and non-selected drug containers is settled
   (3) Means for transmitting and receiving processing information regarding separation and collection of the non-selected drug container among each terminal of medical workers.
[19] A computer program for administration of the kit product according to [10] wherein the computer system includes a data storage area which is connected to each terminal of medical workers and a data storage unit where the medical workers store each the addresses of the terminals thereof, the data storage area is characterized in including the following means (1) to (3) via computer network:
   (1) Means for a doctor to transmit a medication prescription relating to the kit product according to [6], and to transmit and receive the data of the medication prescription among the terminals of medical workers other than the doctor.
   (2) Means for transmitting or receiving the data regarding the selected drug containers and the information thereof and the data regarding the non-selected drug containers and the information thereof, and/or the data on a container containing a diluted or dissolved solution including an infusion solution or pure water and plural drug-enclosing portions having sealing part(s) among each terminal of medical workers in case of the kit product according to [6] based on the data of the medication prescription, classifying selected drug containers and non-selected drug containers is settled
   (3) Means for transmitting and receiving processing information regarding separation and collection of the non-selected drug container among each terminal of medical workers.

Next, various terms used in this patent description will be explained.

"Active ingredients of drugs" means "active ingredients" in the package insert of prescription drugs published by the Agency for Inspecting Drugs and Medical Devices such as the Pharmaceuticals and Medical Devices Agency (PMDA) and medical devices. It means the substances described in "Physical and Chemical Findings".

The "drug usage" is the method of administering the drug described in the "dose" of the prescription package insert of the prescription drug published by PMDA, and posted in Drugs^{@}FDA: FDA Applied Drugs published by FDA. It means the administration method of the drug described in Label's Dosage AND ADMINISTRATION.

"Drug dose" means the dose of the drug described in "Usage Dose" of the package insert of the prescription drug published by PMDA, and posted in Drugs^{@}FDA: FDA Applied Drugs published by FDA. It means the dose etc. of the drug described in Label's Dosage AND ADMINISTRATION.

"Drug manufacturing date" means the date on which the formulation was manufactured.

By "drug expiration date" means the last day on which drugs may be administered to patients.

The "drug shipping date" means the date on which the drugs are shipped from the manufacturing plant.

The "drug delivery route" means a delivery route of the drug delivered from a manufacturing factory and delivered through various people, companies, organizations, etc. before being administered to a patient.

"Traceability of storage state" means that information on environmental conditions (such as temperature, light (dark and light), humidity, and time) that affects the stability of the drug is continuously or discontinuously in the delivery route of the drug is recorded.

"Date of administration of the drug" means the date on which the drug is administered to the patient.

"Details of administration" means the actually administered drug and its amount, the presence or absence of use in a vial, and the used amount when used.

"Amount of drug present in drug enclosing" means the amount or estimated value of the active ingredient of the drug present in drug enclosing portion.

"Patient information" means the height, weight, body surface area, age, renal function, liver function, side effect occurrence status of the administered drug, patient's physical condition, and other information necessary to determine the dose of the drug.
"A drug whose dosage is determined based on patient information" means a drug which has been described to determine a dose using patient information such as mg/kg or mg/m2 described in the dosage and dosage section of the package insert (Label).
"Based on the unit of mg/kg or mg/m2" means that kg represents the dose (mg) of the drug per 1 kg of the patient's body weight, and m2 represents the dose (mg) of the drug/m2 of the body surface area of the patient. It means to determine the dose of the patient based on these.

"Surplus drug" means an unadministered drug calculated after the dose is determined based on dose adjustment and calculated by subtracting the dose from the specified dose of the drug.

"Recovery" means storing the surplus discarded drug for treatment of a patient.

"Reuse method" means the use of surplus drug to formulate a dosage form for treatment of a patient.

The "medical provider" means a doctor who examines and diagnoses and prescribes a drug, or a person who performs a treatment such as drug administration.

The "dispensing provider" means a person who dispenses, and the "pharmaceutical provider " means a person who assists the dispensing provider, for example, a so-called front pharmacy.

"medical worker" refers to doctors who are the above-mentioned drug providers, the above-mentioned dispensing providers and drug providers, as well as drug manufacturers such as drug manufacturing plants (pharmaceutical companies) and/or drug suppliers such as drug wholesalers. means.

The "data storage area" means an information storage medium which includes, for example, traceability of drug dose to a patient, administration schedule, patient information, storage state of the drug to be administered (eg, temperature, light (dark/light), humidity, time, etc.). It mainly refers to the information recording medium on the server, but is not limited to this, and includes an information recording medium of a new technology which will be replaced by the function of the server in the future.

The "drug manufacturer" is the entity that manufactures the final product of a pharmaceutical product.
And it means a person, a company, an organization, or the like.
The "drug supplier" means a person who assists sales of drug manufacturers which includes such as so-called drug wholesalers.

"Medication prescription" refers to the prescription of a drug administered to a patient.

The term "selected packaging container" means all drug-enclosing portions such as ampules, vials, syringes, prefilled syringes, bags, bottles, etc. used for drug preparation.
A vial, a syringe and a prefilled syringe are preferable, a vial and a prefilled syringe are more preferable, and a vial is preferable among them.

The term "non-selected packaging container" means all drug-enclosing portions that are not used for preparation, that is, unused ampoules, vials, syringes, prefilled syringes, bags, bottles and the like.
A vial, a syringe and a prefilled syringe are preferable, a vial and a prefilled syringe are more preferable, and a vial is particularly preferable.

The "dose that requires dose adjustment" means a drug whose dose may be different for each patient as described below.
(1) "Dosage" column of the prescription package for prescription drugs published by the Pharmaceuticals and Medical Devices Agency (PMDA), FOOD AND DRUG ADMINISTRATION (FDA), etc.
(2) Drugs^{@}FDA: Description of mg/kg or mg/m2 etc. in the column of Label of "Dosage AND ADMINISTRATION" described in FDA Applied Drug Products

"High-risk drug" refers to the following drug:
1.
   (1) Drugs that require careful attention to dosage
   (2) Drugs with a drug holiday or drugs requiring management of the medication period
   (3) Drugs that require caution in contraindications and interactions with many drugs
   (4) Pharmaceuticals that are contraindicated in certain diseases and pregnancy
   (5) Pharmaceuticals that require regular examination to avoid serious side effects
   (6) Drugs that require attention for cardiac arrest
   (7) Injection preparations requiring caution in respiratory depression
   (8) Injection preparations whose dose is set in units (Unit)
   (9) Injection preparations that cause skin damage due to leakage
2.
   (1) Anti-neoplastic drug
   (2) Immunosuppressant
   (3) Antiarrhythmic drug
   (4) Antiepileptic drug
   (5) Blood coagulation inhibitor
   (6) Digitalis preparation
   (7) Theophylline preparation
   (8) Potassium preparation
   (9) Psychiatry and nerve agents
   (10) Diabetes agent
   (11) Pancreatic hormone agent
   (12) Anti-HIV agent
3.
   (1) Drugs with a narrow therapeutic range
   (2) Drugs for which it is difficult to control the administration method and dose because the poisoning area and effective area are close
   (3) Drugs with large individual differences in pharmacokinetics
   (4) Drugs with large individual differences due to physiological factors (liver disorders, renal disorders, elderly people, children, etc.)
   (5) Drugs that may cause serious harm to patients due to improper use
   (6) Drugs for which many medical accidents and incidents have been reported
   (7) Other drugs that are strongly required to be used properly

"Hazardous Drug" is a drug having the following characteristics.
1. carcinogenic
2. Teratogenic or developmental toxicity
3. Reproductive toxicity
4. Organ toxicity at low doses
5. Genotoxicity
6. Chemical structure and toxicity profile similar to existing drugs identified as harmful according to 1-5 criteria
Also, "Hazardous Drug" may be a drug having the following characteristics.
1. Carcinogenicity reported by IARC in animal models, patients, or both
2. Teratogenicity in patients undergoing animal studies or treatment
3. Reproductive toxicity in animal experiments or in treated patients
4. Severe organ toxicity and other toxicities at low doses in animal models or in treated patients
5. Genotoxicity (ie mutagenicity and chromosomal aberration in short-term studies)

### Advantageous Effects of Invention

According to the present invention, not only a medical worker is free from exposure to a drug, but also the dose of the drug can be adjusted accurately, so that a medical accident or malpractice can be prevented.
Furthermore, by using the kit product of the present invention and properly managing the kit product, it is possible to prevent the surplus drug from being discarded.
In addition, by recovering and reusing unused medicines for drug administration, it is possible to reduce disposal costs and recover and reuse medicines that should be discarded.
Therefore, it is possible to reduce the drug cost and contribute to the medical economy.

### Description of Embodiments

The first embodiment of the present invention is a packaging container for the drug.
Further, it is characterized by having a recording medium and/or an identification mark for recording/updating one or more arbitrary information selected from the group consisting of the following.
(1) An active ingredient of drug
(2) Usage of a drug
(3) Drug dose
(4) Date of manufacture of a drug
(5) Expiration date of a drug
(6) Drug shipping date of a drug
(7) Traceability of drug delivery route from manufacturing to drug usage and storage conditions including storage temperature and its changes (eg temperature, light (dark and light), humidity, time, etc.)
(8) Information including the administration date of the drug and a breakdown of the administration
(9) Amount of drug present in drug-enclosing portion

The second embodiment is a drug container containing a drug, particularly a drug whose dosage for a patient is determined based on the unit of mg/kg or mg/m2 in terms of usage/dose.

A third embodiment is a kit product consisting of:
(1) Container containing diluted/dissolved solution containing infusion solution or pure water
(2) A plurality of drug-enclosing portions having sealing parts
   (Here, the medicine is enclosed in the packaging container of the first embodiment, and the drug container in which the medicine is enclosed in the packaging container and the container are connected via the sealing part, by opening the sealing part, the container and the drug container communicate with each other)
(3) A series of drug containers according to the second embodiment containing the drug

Among them, a kit product having the following characteristics is preferable.
(1) Kit product in which the above-mentioned drug can be prepared by being placed in a container containing a diluted/dissolved solution
(2) The dose of the drug is selected based on patient information.
(3) It is possible to adjust the dosage of the drug based on the number of significant figures of the dose by selecting the drug and the drug enclosing portion.
(4) It has a composition selected from the group consisting of a drug to be administered and a drug to be separated and not administered.

In particular, a kit product consisting of the following is preferable.
(1) Container containing diluted/dissolved solution containing infusion solution or pure water
(2) A plurality of drug enclosing portions having sealing parts
(3) A plurality of drug containers selected from a series of drug containers
However, in that case, a kit product as a so-called fixed-dose drug can be prepared without the generation of a drug to be separated and not administered in some cases.

The fourth embodiment relates to a business model using the kit product of the third embodiment. A method of managing a kit product including a selected drug container and a non-selected drug container are characterized by including the following configurations (1) to (7):
(1) Step of providing medication prescription
(2) Step of providing the kit product of the third embodiment based on the medication prescription
(3) Step of providing a drug container selected for preparation of the kit product
(4) A drug management method including a step of providing a non-selected drug container for dispensing the kit product
(5) Classification of selected drug containers and non-selected drug containers in the above kit product
(6) Providing data on the selected drug container and its information based on the above classification results
(7) Providing data on non-selected drug containers and their information based on the above classification results
In business model A, a computer control method having the following features 1) and 2) is preferable.
1) The data storage area must include the following configurations (1) to (8).
2) Management of selected drug container and its drug and non-selected drug container and its drug
   (However, the business model A is a business model using a kit product. And, above all, it is a computer system including the following configurations <1> and <2> via a computer network.
   <1> Data storage area connected to the terminal of the medical provider and the terminal of the dispensing provider
   <2> Data storage unit of drug manufacturer and/or drug supplier storing terminal address of drug manufacturer and/or drug supplier)
      (1) Step of accepting medication prescription data from the terminal of the medical provider
      (2) A step of receiving the data of the kit product according to [6] from the terminals of the drug manufacturer and/or the drug supplier based on the data of the medication prescription.
      (3) A step of receiving the data of the drug container selected for dispensing of the kit product at the terminals of the dispensing provider, the pharmaceutical provider, and the drug manufacturer and/or the drug supplier.
      (4) A method of controlling a computer including a step of receiving data of a non-selected drug container for dispensing the kit product at a terminal of the dispensing provider, the pharmaceutical provider, and the drug manufacturer and/or the drug supplier for dispensing.
      (5) Classification of selected drug containers and non-selected drug containers in the above kit product
      (6) Accepting data on the selected drug container and its information based on the above classification result at the terminals of the dispensing provider, pharmaceutical provider and drug manufacturer and/or drug supplier.
      (7) Based on the above classification result, the data related to the non-selected drug container and its information is accepted at the terminal of the dispensing provider, the pharmaceutical provider, and the drug manufacturer and/or the drug supplier. According to this acceptance, non-selected drug containers are separated and recovered.
      (8) Sending the processing information regarding the separation and recovery to the terminal of the medical provider, the terminal of the dispensing provider, and the terminal of the pharmaceutical provider.

The drug management method using this computer control method is characterized by comprising the following (1) to (5).
And, it is a method which can provide management of not only the selected drug container and its medicine but also the non-selected drug container and its drug.
(1) Means for accepting medication prescription data from the terminal of the medical provider
(2) A means for classifying the selected drug container and the non-selected drug container, and receiving the selected drug container and the data related to the information and the non-selected drug container and the data related to the information based on the data of the medication prescription in the above-mentioned kit product.
(3) A means of sending the data regarding the selected drug container and its information and the data regarding the non-selected drug container to be recovered and its information to the terminals of the drug manufacturer and/or drug supplier based on the classification,
(4) A means for recovering the non-selected drug container in response to receiving that the non-selection for the recovery has been received from the terminal of the drug manufacturer and/or the drug supplier.
(5) A means for transmitting processing information for the recovery to the terminal of the medical provider and the terminal of the dispensing provider

The fifth embodiment relates to a computer system having the following features.
<1> Via computer network
<2> Consisting of a data storage area connected to the terminal of the medical provider, the terminal of the dispensing provider, and the terminal of the pharmaceutical provider and data storage unit of drug manufacturer and/or drug supplier storing terminal addresses of drug manufacturer and/or drug supplier
<3> The data storage area must include the following steps of (1) to (8).
<4> Management of selected drug container and its drug and non-selected drug container and its drug
   (1) A step of accepting medication prescription data from the terminal of the medical provider
   (2) A step of receiving the data of the kit product according to [6] from the terminal of the drug manufacturer and/or the drug supplier based on the data of the medication prescription.
   (3) A step of receiving data of a drug container selected for dispensing of the kit product at a terminal of a dispensing provider, a pharmaceutical provider, and a drug manufacturer and/or a drug supplier
   (4) Computer system including a step of receiving data of a non-selected drug container for dispensing of the kit product at a terminal of the dispensing provider, the pharmaceutical provider, and the drug manufacturer and/or the drug supplier
   (5) Classification of selected drug containers and non-selected drug containers in the above kit product
   (6) Accepting data on a selected drug container and its information based on the above classification result at the terminals of the dispensing provider, the pharmaceutical provider, and the drug manufacturer and/or the drug supplier.
   (7) Based on the above classification result, separation and recovery of the non-selected drug container in response to receiving data regarding the drug container and its information at the terminals of the dispensing provider, the pharmaceutical provider, and the drug manufacturer and/or drug supplier.
   (8) To transmit the processing information regarding the separation and recovery to the terminal of the medical provider, the terminal of the dispensing provider, and the terminal of the pharmaceutical provider.

This computer system is a drug management method system having the following <1> to <4> characteristics.
<1> Via computer network.
<2> Consisting of a data storage area connected to the terminal of the medical provider and the terminal of the dispensing provider and data storage unit of drug manufacturer and/or pharmaceutical provider storing terminal addresses of drug manufacturer and/or drug supplier
<3> The data storage area is composed of the following means of (1) to (5).
<4> Not only the selected drug container and its medicine but also the non-selected drug container and its medicine are managed.
   (1) Means of accepting medication prescription data from the terminal of the medical provider
   (2) Means of classifying selected drug containers and non-selected drug containers and accepting data relating to selected drug container and its information and data relating to non-selected drug container and its information based on the data of the medication prescription in the kit product according to [6].
   (3) Means of sending data of the selected drug container and its information and data on the non-selected drug container to be recovered and its information based on the classification to the terminals of the drug manufacturer and/or drug supplier.
   (4) Means of recovering the non-selected drug container in response to receipt of the acceptance of the non-selection/recovery from the terminal of the drug manufacturer and/or the drug supplier
   (5) Means of transmitting the processing information for the non-selection/recovery to the terminal of the medical provider and the terminal of the dispensing provider

The sixth embodiment relates to a computer program having the following features <1> to <4>.
<1> Via computer network.
<2> A computer system including the following 1) and 2).
   1) Data storage area connected to the terminal of the medical provider, the terminal of the dispensing provider, and the terminal of the pharmaceutical provider
   2) Data storage unit of the drug manufacturer and/or drug supplier that stores the terminal addresses of the drug manufacturer and/or drug supplier
<3> The data storage area includes the following steps of (1) to (8).
<4> The selected drug container and its drug, and the non-selected drug container and its drug are managed.
   (1) Step of accepting medication prescription data from the terminal of the medical provider
   (2) A step of receiving the data of the kit product according to [6] from the terminal of the drug manufacturer and/or the drug supplier based on the data of the medication prescription.
   (3) A step of receiving data of a drug container selected for dispensing of the kit product at a terminal of a dispensing provider, a pharmaceutical provider, and a drug manufacturer and/or a drug supplier
   (4) A computer program including a step of receiving data of a non-selected drug container for dispensing of the kit product at a terminal of the dispensing provider, the pharmaceutical provider, and the drug manufacturer and/or the drug supplier.
   (5) Classification of selected drug containers and non-selected drug containers in the above kit product
   (6) Accepting data of the selected drug container and its information based on the above classification result at the terminals of the dispensing provider, the pharmaceutical provider, and the drug manufacturer and/or the drug supplier.
   (7) Separation and recovery of the non-selected drug container according to acceptance of data on non-selected drug containers and their information based on the above classification result, at the terminals of the dispensing provider, the pharmaceutical provider, and the drug manufacturer and/or drug supplier.
   (8) To transmit the processing information regarding the separation and recovery to the terminal of the medical provider, the terminal of the dispensing provider, and the terminal of the pharmaceutical provider.

The seventh embodiment is a method for producing a kit product including a series of drug containers, which utilizes the following <1> to <3>.
<1> Preparing a series of drug containers according to the second embodiment.
   However, it includes the recovered non-selected packaging container described in the fourth embodiment (4), the fifth embodiment (4) and/or the sixth embodiment (4).
<2> A kit product is prepared by combining a plurality of drug-enclosing portions having a container containing diluted/dissolved solution containing the infusion solution or pure water and a sealing part described in the third embodiment, and the series of drug containers.
<3> The drug management method according to the fourth embodiment, the fifth embodiment, or the sixth embodiment

The kit product of the present invention is manufactured by the usual methods 1) and 2) below.
1) Producing a set of kit product by combining drug containers such as diluted/dissolved solution containing infusion or pure water and a series of vials containing the same drug.
2) Store together with the package insert, for example, in one packaging box.

The eighth embodiment is a process for producing a kit product consisting of a series of drug containers having the following features 1) to 4).
1) Include a recovered non-selected packaging container as described in the fourth embodiment (4), the fifth embodiment (4) and/or the sixth embodiment (4).
2) Prepare the series of drug containers described in the second embodiment.
3) A kit product is prepared by combining a plurality of drug-enclosing portions having sealing parts and a container containing the diluted/dissolved solution containing the infusion solution or pure water described in the third embodiment and the series of drug containers.
4) The drug management method described in the fourth embodiment, the fifth embodiment, or the sixth embodiment is used.
Specifically, it means a process consisting of the following 1) to 3).
1) A process of storing each member in a container for packaging a kit product such as a cardboard box, a container for an infusion solution bag with a connection port, and a container for a plurality of independent drug vials.
2) A process of transporting each member independently
3) A process of installing the infusion solution bag and the drug vial in place on the packaging container for the carried kit product.
In this process, a process of confirming the inclusion of package inserts necessary for the product and the reliable installation of the components of the kit product (the infusion bag and the drug vial) can be added.
It should be noted that it is desirable to add a process to remove the missing kit product from the manufacturing process.

A method for recovering and/or reusing a surplus drug, characterized by combining the following 1) and 2):
1) The ninth embodiment is a drug management method according to any of the fourth, fifth, and sixth embodiments.
2) Manufacturing method described in the seventh embodiment.

The tenth embodiment is the kit product management system according to [6], which has the features 1) to 2).
1) A drug management method in a computer system including a data storage area connected to a terminal of each medical worker via a computer network and a data storage unit storing a terminal address of each medical worker
2) The data storage area includes the following means (1) to (3)
   (1) Means for the doctor to transmit the medication prescription relating to the kit product described in [6], and to transmit and receive the data of the medication prescription between the terminals of the medical worker other than the physician.
   (2) Means of sending and receiving the data related to the following A) to C) between the terminals of each medical worker after being classification of the selected drug container and the non-selected drug container on the basis of the medication prescription in the kit product described in [6].
      A) Data on selected drug container and its information
      B) Data on non-selected drug containers and their information
      C) A plurality of drug-enclosing portions having a sealing part and a container that contains a diluted/dissolved solution containing infusion solution or pure water.
   (3) Means for transmitting and receiving the processing information regarding the recovery of the non-selected drug container between the terminals of each medical worker

The eleventh embodiment is the method for managing the kit product according to [6], which is characterized by the following A) to C).
A) Via computer network
B) The present invention relates to a drug management method in a computer system including a data storage unit connected to each medical worker's terminal and a data storage unit storing each medical worker's terminal address.
C) The data storage area includes the following means (1) to (3)
   (1) Means of transmitting the medication prescription relating to the kit product described in [6] by doctors, and sending and receiving the data of the medication prescription between the terminals of the medical worker other than the physician.
   (2) Means of classifying selected drug containers and non-selected drug containers and sending/receiving the following 1) to 3) data between terminals of each medical worker based on the data of the medication prescription, in the kit product according to [6].
      1) Data on selected drug container and its information
      2) Data on non-selected drug containers and their information
      3) Data on a plurality of drug-enclosing portions having a container and a sealing part containing a diluted/dissolved solution containing an infusion solution or pure water.
   (3) Means of sending and receiving the processing information regarding the recovery of the non-selected drug container between the terminals of each medical worker

### Examples

Examples of the packaging container, the drug container in which the drug is enclosed, and the kit product of the present invention are shown below to specifically describe the present invention, but the present invention is not limited thereto.

### Example 1

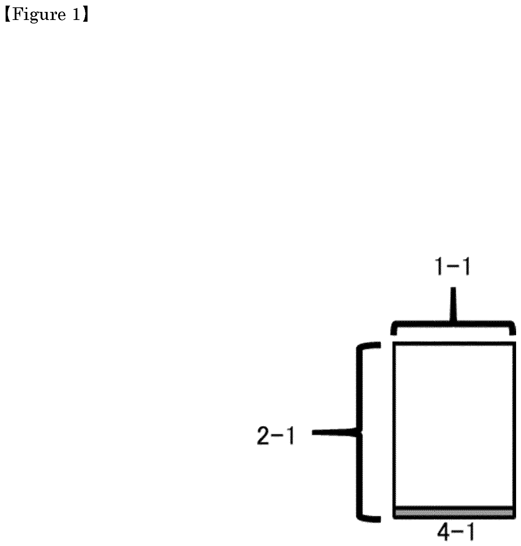

As shown in FIG. 1, a cross-sectional view of a packaging container is shown, 1-1 indicates a vial, 2-1 indicates a vial body (packaging container), 3-1 indicates a drug enclosing part, 4 -1 indicates a septum.
Although not shown, the characteristic feature of the present packaging container is that it has a recording medium and/or an identification mark having the following characteristics A).
A) A recording medium and/or an identification mark for recording/updating one or more arbitrary information selected from the group consisting of the following (1) to (9) on the bottom surface or the side surface of the septum or the packaging container.
   (1) An active ingredient of drug
   (2) Usage of a drug
   (3) Drug dose
   (4) Date of manufacture of a drug
   (5) Expiration date of a drug
   (6) Drug shipping date of a drug
   (7) Traceability of drug delivery route from manufacturing to drug usage and storage conditions including storage temperature and its changes (eg temperature, light (dark and light), humidity, time, etc.)
   (8) Information including the administration date of the drug and a breakdown of the administration
   (9) Amount of drug present in drug-enclosing portion
Furthermore, in addition to (10) the information on the type of drug to be administered and the type of drug to be separated and not administered, information necessary for managing the drug can be appropriately recorded/updated in the packaging container.

### Example 2

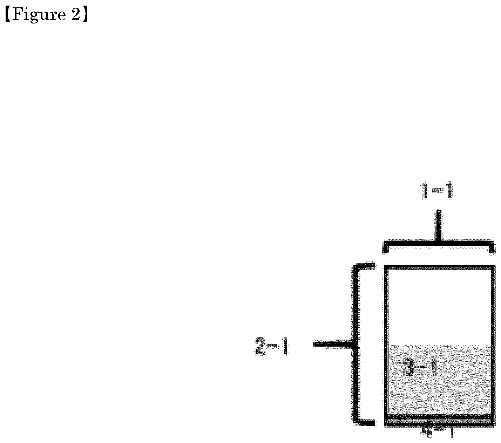

As shown in FIG. 2, a sectional view of a packaging container containing a drug is shown, 1-1 is a vial, 2-1 is a vial body (packaging container), and 3-1 is an encapsulated drug. And 4-1 represents a septum.
Although not shown, the characteristic feature of the present packaging container is to have a recording medium and/or an identification mark with one or more arbitrary information selected from the group consisting of the following (1) to (9) which is recorded/updated on the bottom surface or the side surface of the septum or the packaging container.
(1) An active ingredient of drug
(2) Drug usage
(3) Drug dose
(4) Date of manufacture of a drug
(5) Expiration date of a drug
(6) Drug shipping date of a drug
(7) Traceability of drug delivery route from manufacturing to drug usage and storage conditions including storage temperature and its changes (eg temperature, light (dark and light), humidity, time, etc.)
(8) Information including the administration date of the drug and a breakdown of the administration
(9) Amount of drug present in drug-enclosing portion
Furthermore, in addition to (10) the information on the type of drug to be administered and the type of drug to be separated and not administered, information necessary for managing the drug can be appropriately recorded/updated in the packaging container.

### Example 3

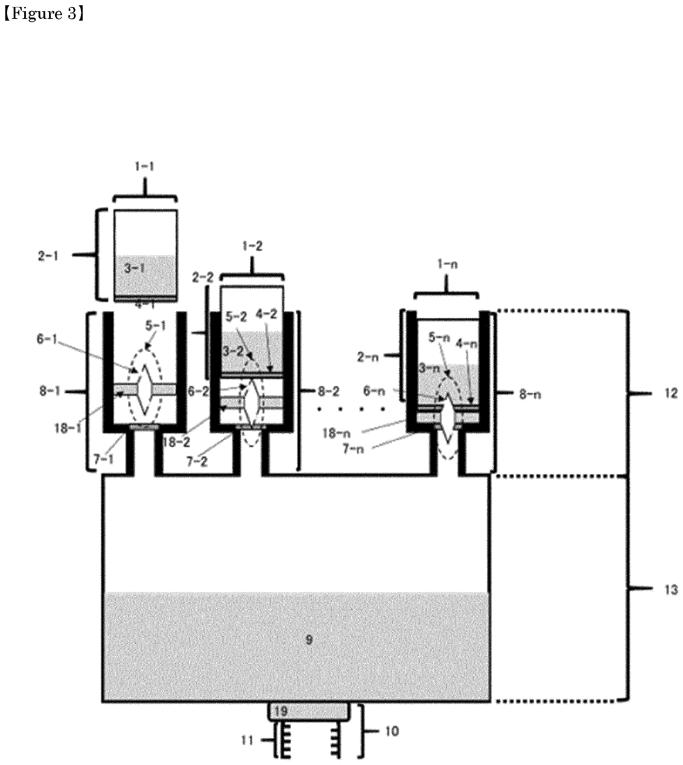

As shown in FIG. 3, there is shown a kit product comprising a container accommodating a diluted/dissolved solution containing an infusion solution or pure water, a plurality of drug-enclosing portions having a sealing part, and a series of drug containers containing the drug. With respect to the container containing the diluted/dissolved solution containing the infusion solution or pure water, the drug enclosing portion and the drug container containing the drug are plural to 100, preferably 20, more preferably 10, and further preferably 5.
1-1, 1-2,..., 1-n represent vials,
2-1, 2-2,..., 2-n respectively indicate drug-enclosing portions,
3-1, 3-2,..., 3-n respectively represent the drug enclosed in the vial,
4-1, 4-2,..., 4-n respectively represent septum parts forming a part of the vial,
5-1, 5-2,..., 5-n are double-ended needles,
6-1, 6-2,..., 6-n respectively indicate holes formed in the double-ended needles,
7-1, 7-2,..., 7-n respectively indicate septa of connection ports,
8-1, 8-2,..., 8-n respectively indicate connection ports,
9 denotes a diluted/dissolved solution containing an infusion solution or pure water in the infusion solution bag part (13),
10 is a connecting portion for connecting the infusion solution bag part (13) and the administration route (17),
10 shows a septum (19) to which the needle (14) of the administration route is pierced and a female screw structure (11) for fixing the needle (14) so as not to come off,
11 shows a female screw structure for fixing the administration route (17) to the infusion solution bag part (13),
12 indicates a connection port section,
13 shows an infusion solution bag part,
18-1, 18-2,..., 18-n respectively indicate supports for holding the double-ended needles in a position perpendicular to the septum,
19 denotes a septum portion to which the needle (14) of the administration route (17) is stuck.

### Example 4

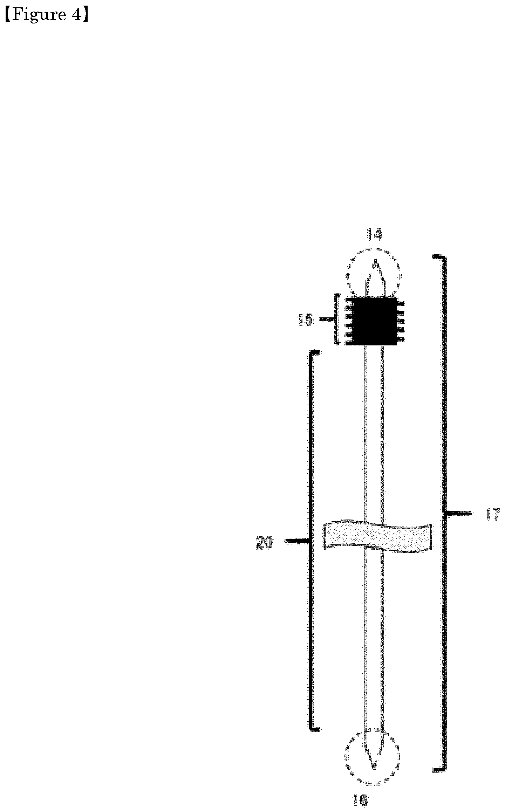

As shown in FIG. 4, the administration route connecting to the kit product of FIG. 3 is shown, and 14 needles are connected to 19 septa via 11 female screw structure and 15 male screw structure parts.
14 denotes a needle that pierces the infusion solution bag part (13) of the administration route (17),
15 shows a male screw structure for fixing the administration route (17) to the infusion solution bag part (13),
16 shows the administration route (17) and a needle for piercing the blood vessel of the patient,
17 indicates the administration route,
20 shows a tube structure forming a part of the administration route (17).

In addition, in the kit product of the present invention, the number of connection ports (pieces) related to dose adjustment, the number of surplus ports (pieces), the number of vials (pieces), the number of vials per digit of the dose (pieces), and the relationship of the type and the numbers (pieces) of standard is described as in Table 1 below.

**[table 1]**

| Number of connection ports | | | Vials used for dose adjustment | | |
|---|---|---|---|---|---|
| total | Used for dose adjustment | Not used for dose adjustment | number | type(mg) | Number of standards |
| 1 | 1 | 0 | 100 | 1, 2, 3···100 | 100 |
| 2 | 2 | 0-1 | 18 | 1, 2, 3, 4, 5, 6, 7, 8, 9 | 18 |
| | | | | 10, 20, 30, 40, 50, 60, 70, 80, 90 | |
| 3 | 3 | 0-2 | 18 | 1, 2, 3, 4, 5, 6, 7, 8, 9 | 18 |
| | | | | 10, 20, 30, 40, 50, 60, 70, 80, 90 | |
| 4 | 4 | 0-3 | 10 | 1, 2, 3, 4, 5, 10, 20, 30, 40, 50 | 10 |
| 5 | 5 | 0-4 | 10 | 1, 2, 3, 4, 5, 10, 20, 30, 40, 50 | 10 |
| 6 | 6 | 0-5 | 8 | 1, 2, 3, 4, 10, 20, 30, 40 | 8 |
| 7 | 7 | 0-6 | | or | |
| 8 | 8 | 0-7 | | 1, 2, 3, 5, 10, 20, 30, 50 | |
| 9 | 8 | 1-8 | | or | |
| | | | | 1, 2, 3, 6, 10, 20, 30, 60 | |
| 10 | 8 | 2-9 | | or | |
| 20 | 8 | 12-19 | | 1,2,3,7, 10, 20, 30, 70 | |

When the number of connection ports is 1, the number of connection ports used for dose adjustment is 1, and the number of connection ports not used for dose adjustment is 0.
At this time, the number of vials required is 100, and the types thereof are 1 mg, 2 mg, 3 mg,... 100 mg, and the number of standards is 100.

When the number of connection ports is 2, the maximum number of connection ports used for dose adjustment is 2, and the number of connection ports not used for dose adjustment is 0 to 1.
At this time, the number of vials required is 18, the types thereof are 1 mg, 2 mg,... 9 mg, 10 mg,... 90 mg, and the number of standards is 18.

When the number of connection ports is 3, the maximum number of connection ports used for dose adjustment is 3, and the number of connection ports not used for dose adjustment is 0 to 2.
At this time, the number of vials required is 18, the types thereof are 1 mg, 2 mg,... 9 mg, 10 mg,... 90 mg, and the number of standards is 18.

When the number of connection ports is 4, the maximum number of connection ports used for dose adjustment is 4, and the number of connection ports not used for dose adjustment is 0 to 3. At this time, the number of vials required is 10, and the types thereof are 1 mg, 2 mg,... 5 mg, 10 mg,... 50 mg, and the number of standards is 10.

When the number of connection ports is 5, the maximum number of connection ports used for dose adjustment is 5, and the number of connection ports not used for dose adjustment is 0 to 4.
At this time, the number of vials required is 10, and the types thereof are 1 mg, 2 mg,... 5 mg, 10 mg,... 50 mg, and the number of standards is 10.

When the number of connection ports is 6, the maximum number of connection ports used for dose adjustment is 6, and the number of connection ports not used for dose adjustment is 0 to 5.
At this time, the number of vials required is 8, and the types are "1 mg, 2 mg, 3 mg, 4 mg, 10 mg, 20 mg, 30 mg, 40 mg" or "1 mg, 2 mg, 3 mg, 5 mg, 10 mg, 20 mg, 30 mg, 50 mg or "1 mg, 2 mg, 3 mg, 6 mg, 10 mg, 20 mg, 30 mg, 60 mg" or "1 mg, 2 mg, 3 mg, 7 mg, 10 mg, 20 mg, 30 mg, 70 mg", and the number of standards is eight.

When the number of connection ports is 7, the maximum number of connection ports used for dose adjustment is 7, and the number of connection ports not used for dose adjustment is 0 to 6.
At this time, the number of vials required is 8, and the types are "1 mg, 2 mg, 3 mg, 4 mg, 10 mg, 20 mg, 30 mg, 40 mg" or "1 mg, 2 mg, 3 mg, 5 mg, 10 mg, 20 mg, 30 mg, 50 mg" or "1 mg, 2 mg, 3 mg, 6 mg, 10 mg, 20 mg, 30 mg, 60 mg" or "1 mg, 2 mg, 3 mg, 7 mg, 10 mg, 20 mg, 30 mg, 70 mg", and the number of standards is eight.

When the number of connection ports is 8, the maximum number of connection ports used for dose adjustment is 8, and the number of connection ports not used for dose adjustment is 0 to 7.
At this time, the number of vials required is 8, and the types are "1 mg, 2 mg, 3 mg, 4 mg, 10 mg, 20 mg, 30 mg, 40 mg" or "1 mg, 2 mg, 3 mg, 5 mg, 10 mg, 20 mg, 30 mg, 50 mg" or "1 mg, 2 mg, 3 mg, 6 mg, 10 mg, 20 mg, 30 mg, 60 mg" or "1 mg, 2 mg, 3 mg, 7 mg, 10 mg, 20 mg, 30 mg, 70 mg", and the number of standards is eight.

When the number of connection ports is 9, the maximum number of connection ports used for dose adjustment is 8, and the number of connection ports not used for dose adjustment is 1 to 8.
At this time, the number of vials required is 8, and the types are "1 mg, 2 mg, 3 mg, 4 mg, 10 mg, 20 mg, 30 mg, 40 mg" or "1 mg, 2 mg, 3 mg, 5 mg, 10 mg, 20 mg, 30 mg, 50 mg" or "1 mg, 2 mg, 3 mg, 6 mg, 10 mg, 20 mg, 30 mg, 60 mg" or "1 mg, 2 mg, 3 mg, 7 mg, 10 mg, 20 mg, 30 mg, 70 mg", and the number of standards is eight.

When the number of connection ports is 10, the maximum number of connection ports used for dose adjustment is 8, and the number of connection ports not used for dose adjustment is 2 to 9.
At this time, the number of vials required is 8, and the types are "1 mg, 2 mg, 3 mg, 4 mg, 10 mg, 20 mg, 30 mg, 40 mg" or "1 mg, 2 mg, 3 mg, 5 mg, 10 mg, 20 mg, 30 mg, 50 mg" or "1 mg, 2 mg, 3 mg, 6 mg, 10 mg, 20 mg, 30 mg, 60 mg" or "1 mg, 2 mg, 3 mg, 7 mg, 10 mg, 20 mg, 30 mg, 70 mg", and the number of standards is eight.

When the number of connection ports is 20, the maximum number of connection ports used for dose adjustment is 8, and the number of connection ports not used for dose adjustment is 12 to 19.
At this time, the number of vials required is 8, and the types are "1 mg, 2 mg, 3 mg, 4 mg, 10 mg, 20 mg, 30 mg, 40 mg" or "1 mg, 2 mg, 3 mg, 5 mg, 10 mg, 20 mg, 30 mg, 50 mg" or "1 mg, 2 mg, 3 mg, 6 mg, 10 mg, 20 mg, 30 mg, 60 mg" or "1 mg, 2 mg, 3 mg, 7 mg, 10 mg, 20 mg, 30 mg, 70 mg", and the number of standards is eight.

Next, a method for managing the kit product of the present invention, particularly a method for managing the kit product via a computer network will be described.

First, the method for managing the kit product of the present invention will be described.
A business model for managing a kit product of the present invention is characterized by including the following (1) to (7), and be able to manage a kit product including a selected drug container and a non-selected drug container.
(1) A step of providing a medication prescription
(2) A step of providing the kit product according to [6] based on the medication prescription.
(3) A step of providing a drug container selected for dispensing of the kit product
(4) A method of managing a drug, which comprises the step of providing a non-selected drug container for dispensing the kit product.
(5) Classification of selected drug containers and non-selected drug containers in the above kit product
(6) To provide data on the selected drug container and its information based on the above classification result.
(7) To provide data on non-selected drug containers and their information based on the above classification results

Among them, the method for managing the kit product through the computer network of the present invention, the method for producing the kit product, and the recovery and/or reuse of the surplus drug through the method for managing the kit product can be performed by a business model according to the description of table 2 below.

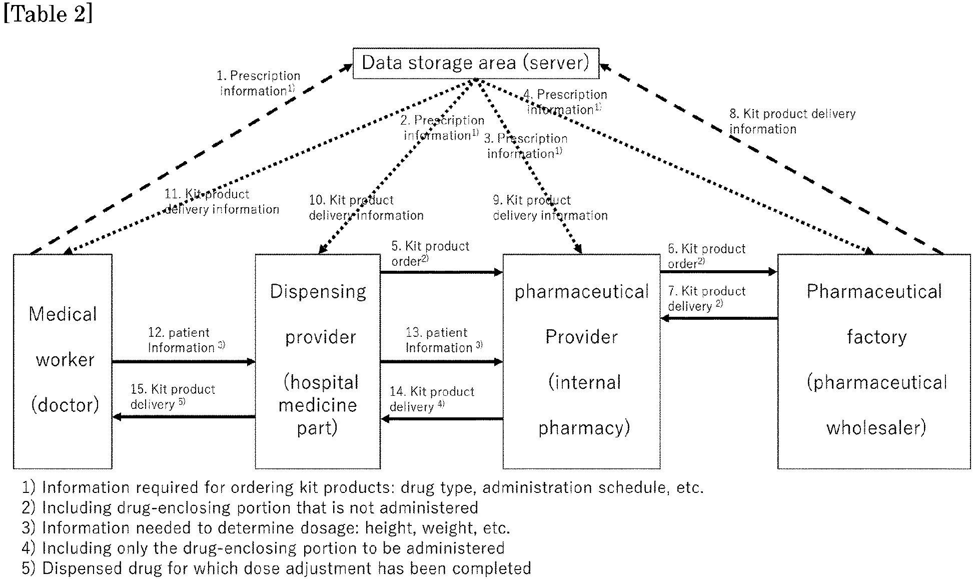

In this business model, (1) a medical provider such as a doctor, (2) a dispensing provider such as a hospital medicine part (pharmacist) or the like, and (3) a pharmaceutical provider such as a medical pharmacist (pharmacist), and (4) a drug supplier such as a drug manufacturer and/or a medicine wholesale of the preparation plant (pharmaceutical company), stores data, information and the like generated in the business in a data storage area of a server or the like, and allows sharing of information through the computer network, and performs business by the following steps (1) to (5)

1)By examining and diagnosing the patient, the medical provider stores the data of the medication prescription, which describes the type of the drug, the dosage of the drug, the administration schedule, etc., in the data storage area for the drug required for the treatment. At the same time, in the case of a hospital, the medical provider provides the dispensing provider with patient information such as the height and weight of the patient necessary to determine the dosage, and the dispensing provider provides the patient information with pharmaceutical provider s.
   Furthermore, the dispensing provider orders the drug manufacturer and/or the drug supplier via the pharmaceutical provider (or directly) to buy the kit product containing the drug enclosing portions that is not administered.
   The drug manufacturer and/or drug supplier delivers the ordered kit product to the pharmaceutical provider (or medical provider or dispensing provider).
   In the case of home medical care, etc., the medical provider directly orders the drug manufacturer and/or the drug supplier for the kit product containing the drug enclosing portion not to be administered, and the drug manufacturer and/or the drug supplier to deliver the kit product ordered by the medical provider.
   Even in the case of a medical practitioner who is in charge of home medical care, when receiving support from a dispensing pharmacy in the neighborhood, an order is placed through the dispensing pharmacy and the kit product is supplied through the dispensing pharmacy.
2) Based on the stored medication prescription data, selected drug containers and non-selected drug containers are classified in the kit product, and data regarding the selected drug container and its information and non-selected drug container and its information is stored in the data storage area.
3) Data regarding the selected drug container and its information and data regarding the non-selected drug container and its information are transmitted to the terminals of medical providers, dispensing providers, pharmaceutical providers and drug manufacturers and/or drug providers, based on the classification.
4) The non-selected drug container is recovered by the drug provider or the dispensing provider or medical provider in response to receiving that the non-selection has been accepted from the terminal of the dispensing provider, the pharmaceutical provider, and the drug manufacturer and/or drug supplier.
   In parallel, the drug provider provides the medical provider with a kit product that matches the medication prescription,
   or the dispensing provider or medical provider directly obtains the kit product,
   the kit product is used for treatment.
5) The dispensing information and the processing information for the recovery are transmitted to the terminal of the medical provider, the terminal of the dispensing provider, the terminal of the pharmaceutical provider, and the terminal of the drug manufacturer and/or the drug supplier.

As an example, if a pharmacy near the medical institution that provides cancer treatment (an internal pharmacy(pharmacies right outside a doctor's office or hospital)) manages the storage of kit products, dispensing support, and management of remaining drugs, the kit product is composed of many vial formulations as its parts. Therefore, the problems (1) securing a storage place at the time of storage, (2) mistakes due to complexity of preparation and (3) complexity of management of remaining drug are solved. Specifically, the kit product is managed according to the following flow.
1) A medical institution places an order for a kit product at the internal pharmacy.
2) The internal pharmacy stores the kit product until it is used.
3) Send the patient data necessary for dispensing to the internal pharmacy immediately before use by the medical institution.
4) Based on the data sent from the medical institutions, the internal pharmacy will deliver the kit product to the medical institutions at the final stage of dosage adjustment (Just piercing the vial).
5) The medical institution performs the final stage of dose adjustment (piercing the vial) and completes the dispensing.
6) The remaining drugs (surplus drugs) generated shall be managed and stored by the internal pharmacy and used appropriately and in a timely manner.
7) Under the current Pharmaceutical Affairs Law, dispensing is limited to pharmacists engaged in hospitals, pharmacies, etc., or those who dispense under the direction of a doctor, but due to deregulation, etc. If the distributor is eased to a distributor such as a wholesaler, the distributor can also manage the regimen kit product.
Alternatively, drug management may be performed by a dispensing provider (pharmacy of a medical institution) or a pharmaceutical provider (internal pharmacy).
Furthermore, in some cases medicines do not go through a drug supplier (distributor), but directly from a drug manufacturer (formulation factory) to a medical provider (doctor of a medical institution), a dispensing provider (pharmaceutical department of a medical institution), a pharmaceutical provider (internal pharmacy).
Alternatively, in the case of home medical care, etc., if the medical provider directly manages the drug, and if the doctor performing the home medical care receives the support of a nearby dispensing pharmacy, the dispensing pharmacy may manage the drug in some cases.
In such cases, drug manufacturers may supply drugs directly to those that manage them.
In this case, the drug supply may or may not be via a drug supplier (distributor).

Next, a computer system and a computer program for executing management of the kit product via a computer network,
method and process for manufacturing the kit product
and the method of recovering and/or reusing the surplus drug through the management method of the kit product will be showed,
and the present invention will be specifically described, but the present invention is not limited thereto.

### Example 5

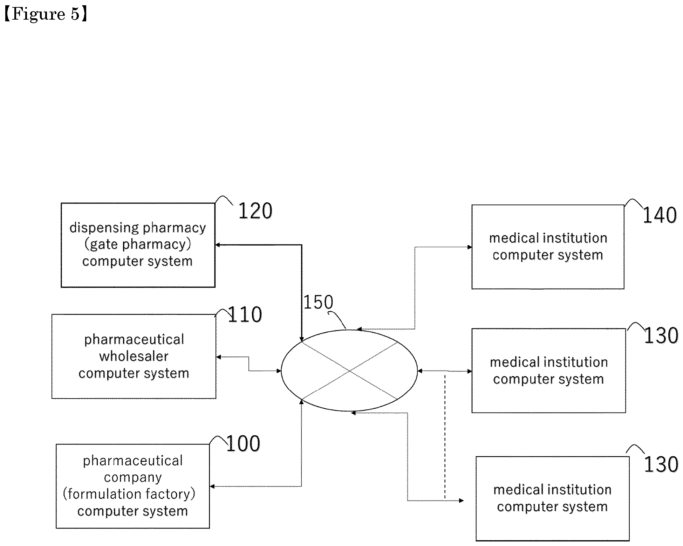

FIG. 5 is a block diagram showing a computer system for executing the method for managing a kit product according to the present invention.
In the figure, 100 is a computer system of a pharmaceutical company (formulation factory), 110 is a computer system of a pharmaceutical wholesaler, 120 is a computer system of a dispensing pharmacy (internal pharmacy), and 130 and 140 are computer system of a medical institution. Reference numeral 150 denotes the Internet (a network that performs data communication in accordance with the communication protocol of TCP/IP protocol (Transmission Control Protocol/Internet Protocol)).
The Internet 150 is shown as a representative example of communication means, and may be ordinary data communication or the like. In the present embodiment, a description will be given using the Internet.
Therefore, the computer system 100 of the pharmaceutical company (formulation factory) or the computer system 110 of the pharmaceutical wholesaler has a WWW server.
And the computer system 120 of the dispensing pharmacy (internal pharmacy), the computer system 130 of the medical institution, and the computer system 140 of the medical institution, which are the computer systems of those customers, have WWW browsers.
In addition, it is also possible to implement a mode in which a WWW browser is used in each computer system by using a cloud server.
Further, 150 may have other communication means not via the Internet, for example, a dedicated line may be installed, or a shared network other than the Internet may be used. Also, 110 (pharmaceutical wholesaler) or 120 (dispensing pharmacy (internal pharmacy)) may not enter the network.

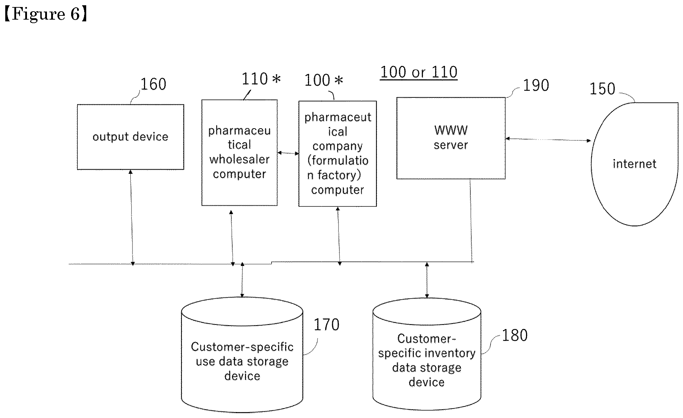

The computer system of this embodiment is
A pharmaceutical wholesaler collects data and related information transmitted from computer systems 120, 130, 140 of a dispensing pharmacy or a medical institution (customer).
It is taken into the computer system 100 or the computer system 110 via the WWW server (including a mail server etc.) 190 (see FIG. 6) via the Internet 150.
Then, according to the prescription by the doctor, the selected drug container and its drug and the non-selected separated and recovered drug container and its drug are classified,
This is a system that manages data related to selected drug containers and their information and data related to non-selected drug containers and their information based on the classification result (where the non-selected drug containers follow the prescription, It may not be done.).
Further, the WWW browsers of the computer systems 120, 130, 140 have a function of searching and displaying data of the computer system 100 of a pharmaceutical company (formulation factory) or the computer system 110 of a pharmaceutical wholesaler.

Also, there are various forms of customer scale,
For example, in medical institutions,
Although described as computer systems 130 and 140,
There are a scale capable of processing data such as a receipt and a chart by a single computer (standalone type) and a small scale system, or a scale capable of processing by a client/server type computer system including a LAN.
130 is a small-scale computer system, and 140 is a large-scale computer system.
Similarly, a computer system 120 of a dispensing pharmacy (internal pharmacy) also has a stand-alone type capable of processing and a system having a small LAN.
In the case of using the cloud server, the processing can be performed by each computer system regardless of the various types of customer scales as described above.

The computer 100* of the pharmaceutical company (formulation factory) or the computer 110* of the pharmaceutical wholesaler will be described with reference to FIG 6.
It should be noted that these two computers do not necessarily have to exist as a server function, but in order to enhance management/maintenance, it is preferable to have them as backups.
A computer 100* of a pharmaceutical company (pharmaceutical factory) or a computer 110* of a pharmaceutical wholesaler includes a LAN with a customer-specific use data storage device 170, a customer-specific inventory data storage device 180, an output device 160, etc. It is connected to the Internet 150 via a WWW server 190.
The computer 100* of the pharmaceutical company (pharmaceutical factory) or the computer 110* of the pharmaceutical wholesaler may use individual data processing as a distributed processing type.
And, including such a configuration, it is named a computer for a pharmaceutical company (formulation factory) or a pharmaceutical wholesaler business.
The customer-specific usage data storage device 170 stores the consumed medicine data of each drug, which is the consumed medicine data transmitted from the customer and the related information for each customer.
The customer-specific inventory data storage device 180 corresponds to an inventory master file, and stores the inventory data of each drug for each customer.
The output device 160 is a display or printer such as a CRT (Cathode Ray Tube) or an LC (liquid crystal).

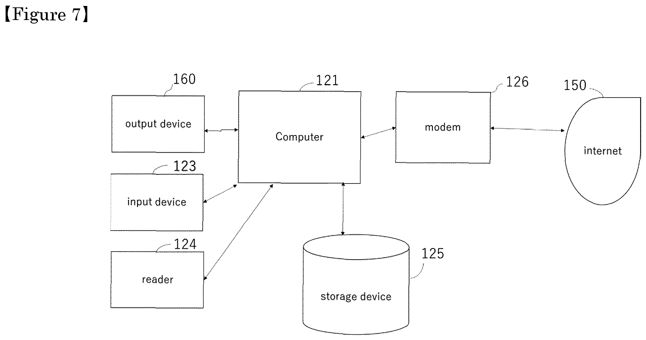

As shown in FIG. 7, a computer system 120 of a dispensing pharmacy (internal pharmacy) is provided with a computer 121, and is connected to an output device 122, an input device 123 such as a keyboard, a reader 124, a storage device 125, and the like. The computer 121 is connected to the Internet 150 via the modem 126. The computer system classifies the drug container and its drug selected in the regimen kit product and the non-selected separated and recovered drug container and its drug with the keyboard that is the input device 123, according to the prescription issued to administer the patient.
Based on the classification result, data regarding a selected drug container and its information and data regarding a non-selected drug container and its information are input to the computer 121.
Then, the patient name, the drug, the data thereof, and the like are stored in the storage device 125, and a monthly medical treatment fee statement (receipt) of the regimen kit product (medicine) for medication is calculated based on the drug price standard. It is output to the output device (display device or printer) 122.
In addition, when paying out the medicine based on the prescription, a monthly receipt is created using the input device 123 according to the following procedure.
1) Prescription drug name, drug name and its quantity (here, the quantity includes data on the selected drug container and its information and data on non-selected drug containers and their information), medical department, etc. are entered.
2) A bill will be issued stating the amount charged for the individual payment of the drug for the patient.
3) Those medication data are stored in the storage device 125.
   The data and the like input from the input device 123 or the reader 124 are converted into a predetermined format and transmitted to the wholesale computer system 110 for pharmaceutical wholesaler.
   Normally, a pharmaceutical company sells a drug through a pharmaceutical wholesaler, and therefore the pharmaceutical wholesaler computer 110 is the basis of a computer system that executes the kit product management method according to the present invention.
   The computer system 100 of a pharmaceutical company (pharmaceutical factory) is useful when a product is sold directly or as a backup of a pharmaceutical wholesaler business computer 110.

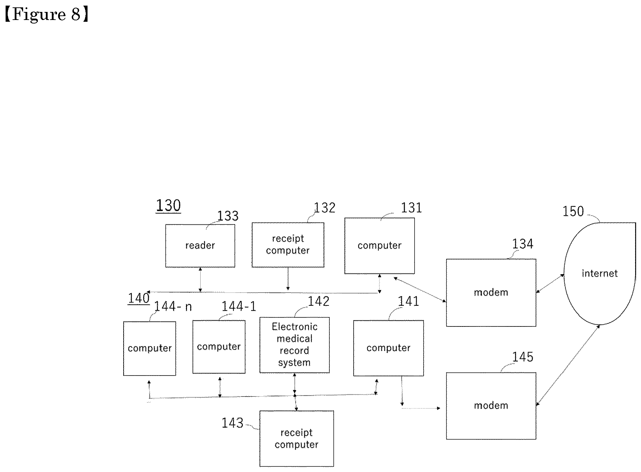

As shown in FIG. 8, medical institutions (customers) have various medical treatment scales. To describe a typical example, in a computer system 130 of a medical institution, a computer 131 is connected to a reception computer 132 and a reader 133 to form a LAN, and is connected to the Internet 150 via a modem 134.
In a computer system 140 of a large-scale medical institution, the computer 141 is connected to the electronic medical chart system 142 or the receipt computer 143, and each doctor terminal (hereinafter, referred to as PC) 44-1 to 44-n is connected. Is configured as a LAN and is connected to the Internet 150 via the modem 145 to configure a medical institution side system. In FIG. 8, the input/output device is omitted.

In the following, referring to FIG. 5 to FIG. 8, in the computer systems 120, 130, 140 of dispensing pharmacies and medical institutions, consumption drug data etc. ("date data", "customer code", "drug code", "Clinical department code", "consumption (including data on selected drug container and its information and data on non-selected drug container and its information)", "drug name", "user code", etc.). It is converted into a predetermined format and transmitted to the business computer system 110 for pharmaceutical wholesaler or the business computer 100 for pharmaceutical factory.
Similarly, the consumed medicine data and the like read by the readers 124 and 133 are converted into a predetermined format and transmitted to the wholesale business computer system 110 on the pharmaceutical wholesaler side or the business computer 100 on the pharmaceutical factory.
Further, the electronic medical chart system 142 includes data such as prescriptions input by the doctor from the PCs 144-1 to 144-n (including data on selected drug containers and their information and data on unselected drug containers and their information). It is input to the electronic medical record system 142, and the consumed drug data and the like are converted into a predetermined format and transmitted to the wholesaler computer system 110 on the pharmaceutical wholesaler side.

On the other hand, the pharmaceutical wholesaler business computer 110 on the pharmaceutical wholesaler side or the business computer 100 on the drug product factory is connected to the computer 121 of a plurality of dispensing pharmacies and the computer 131 and 141 of the computer systems 130 and 140 of a plurality of medical institutions via the Internet 150 and constitutes a computer system for executing the management method of the present kit product.
The wholesale business computer 110 or the pharmaceutical factory business computer 100 has a function of receiving drug consumption data and the like transmitted by the dispensing pharmacy computer system 120 and the medical institution computer systems 130 and 140.
These data include the following 1) to 7).
1) "Date"
2) "Customer code"
3) "Pharmaceutical code"
4) "Clinical department code"
5) "Consumption amount (including data on selected drug containers and their information and data on non-selected drug containers and their information)"
6) "Chemical name"
7) "User code" etc.

The pharmaceutical wholesaler business computer 110 or the pharmaceutical factory business computer 100 receives the following data 1) and 2) and stores usage data for each customer in the use data storage device for each customer 170.
1) Consumption drug data transmitted as text sentences from the computer system 120 of each dispensing pharmacy, the computer system 130 of a medical institution, or the computer system 140
   (In addition, the data on the selected drug container and its information and the data on the non-selected drug container and its information are included.)
2) Order data
   ("Date", "customer code", "drug code", "medical department code", "necessary amount (including data on selected drug container and its information and data on non-selected drug container and its information), "drug name", "user code", etc.)
The Customer-specific use data storage device 120 is provided with a storage area for each medical institution and dispensing pharmacy, and the storage area stores data such as 1) to 5) below.
1) "Customer code"
2) "Chemical code"
3) "Clinical department code"
4) "Consumption amount (including data on selected drug container and its information and data on non-selected drug container and its information)"
5) "Chemical name"
In the stored drug consumed data, the number used (amount used) is periodically aggregated for each drug code.
Further, the wholesale computer 110 or the pharmaceutical computer 100 of the pharmaceutical factory is provided with a drug replenishing mode selecting means, and the drug is replenished and requested according to the consumption scale of the customer and the order of the drug. The ordered quantity of medicine is delivered.

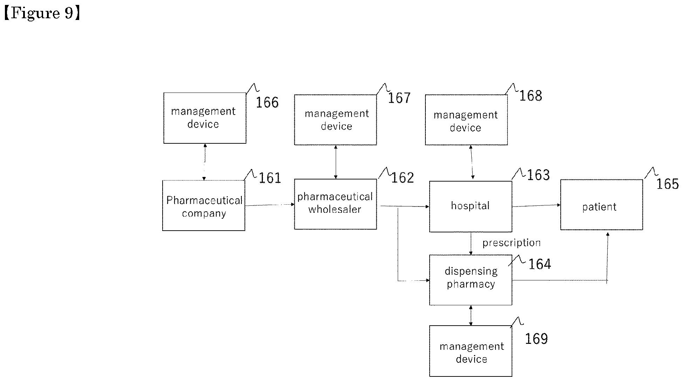

Fig. 9 shows the distribution form of ordinary medicines. The medicine supplied from the pharmaceutical company 161 is wholesaled from the pharmaceutical wholesaler 162 to the hospital 163 or the pharmacy 164, or is directly supplied to the hospital 163 or the pharmacy 164, and is delivered to the consumer (patient) 165 based on the prescription of the doctor.
The pharmaceutical wholesaler 162, the hospital 163, and the pharmacy 164 independently install drug inventory management devices and their systems 166 to 169 (medicine ordering devices and their systems) and manage.
In the hospital 163, when the supply department (medicine department, doctor, nurse, and other management departments) orders medicines, these systems are used to check the current inventory status, grasp the estimated inventory quantity, and place the order. It is a system that decides the quantity and raises an order slip to order a drug.
A pharmaceutical wholesaler or a pharmaceutical company will deliver a drug based on an ordering slip from a hospital (medical institution).
On the other hand, the pharmaceutical wholesaler 162 or the pharmaceutical company 161 operates its own inventory management system to manage it so that it can respond to the customer's request.

### Example 6

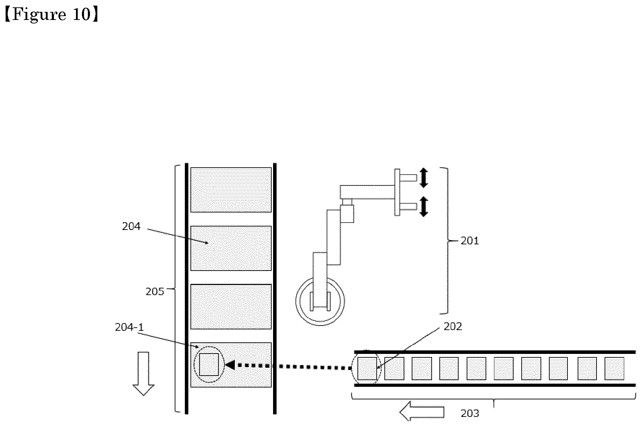

FIG. 10 shows a process diagram for producing the kit product according to the present invention.
Then, FIG. 10 shows a scene in which the infusion solution bag 202 with a connection port is set in a packaging container 204 of a kit product such as a cardboard box by the multi-axis robot arm 201.
Although not shown in the drawing, the kit product packaging container 204 was conveyed from the storage part of the kit product packaging container by the belt conveyor 205 for the kit product packaging container.
Although not shown in the drawing, the infusion bag 202 with a connection port was conveyed from the storage part of the infusion solution bag with a connection port by the belt conveyor 203 of the infusion solution bag with a connection port.
The multi-axis robot arm 201 grabs the infusion bag 202 with a connection port on the belt conveyor 203, installs the infusion bag 202 with a connection port at a predetermined position on the container 204 for packaging the kit product on the belt conveyor 205, and a container 204-1 for packaging the kit product was obtained.

### Example 7

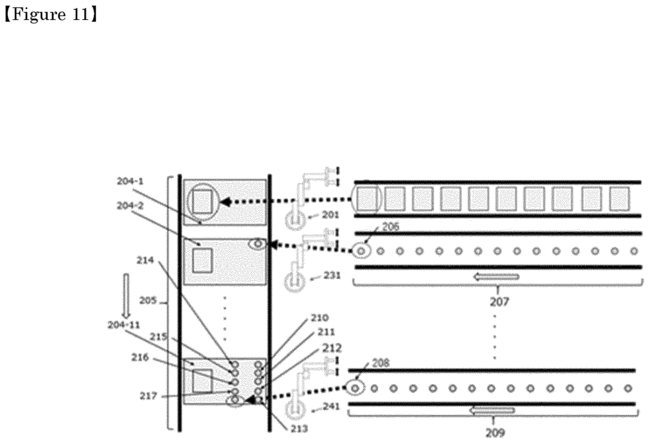

FIG. 11 shows a process diagram for producing the kit product according to the present invention.
Further, FIG. 11 shows a scene in which the infusion bag 202 with a connection port and ten kinds of drug vials (206, 208, 210, 211, 212, 213, 214, 215, 216 and 217) are set, for example, to the packaging container 204 for the kit product such as a cardboard box with the multi-axis robot arm 201.
Although not shown in the drawing, the drug vial 206 was conveyed from the drug vial storage part to the kit product packaging container 204-1 obtained in Example 6 by the drug vial belt conveyor 207.
The multi-axis robot arm 221 picks up the drug vial 206 on the belt conveyor 207 and installs the drug vial 206 on the belt conveyor 205 at a predetermined position of the kit product packaging container 204-1. Then, a container 204-2 for packaging the kit product was obtained. The kit product packaging container 204-11 includes ten kinds of drug vials (206, 208, 210, 211, 212, 213, 214, 215, 216 and 217) at predetermined positions of the kit product packaging container 204-1.
The drug vial 208 on the belt conveyor 209 is installed by the multi-axis robot arm 241.
The drug vials 210, 211, 212, 213, 214, 215, 216 and 217 are installed in a predetermined position on the kit product packaging container 204-11 by a multi-axis robot arm in the same manner.

### Example 8

FIG. 11 shows a plan view of the multi-axis robot arm. The grasping portion 218 is a portion for grasping the infusion bag 202 with a connection port and ten kinds of drug vials (206, 208, 210, 211, 212, 213, 214, 215, 216 and 217), and 19 shows joints of a robot arm. The arrow indicates the range of motion.

### Example 9

FIG. 12 shows a side view of the multi-axis robot arm.
The grip portion 218 is a portion for gripping the infusion solution bag 202 with a connection port and the 10 kinds of drug vials (206, 208, 210, 211, 212, 213, 214, 215, 216 and 217).
220, 221, 222, 223 and 224 denote joints of the robot arm.
The arrow indicates the range of motion.

### Example 10 (Production of kit product)

Doctor A examined patient B and created a prescription to administer 80 mg of drug C that requires dose adjustment.
As for drug C, a 100 mg kit product in which 50 mg, 20 mg, 20 mg and 10 mg vials are combined is sold, and the hospital medicine part where the doctor works orders the kit product to the packaging container through the internal pharmacy.
The packaging container has delivered the 100 mg kit product to the internal pharmacy, and the pharmacist of the internal pharmacy selects vials of 50 mg, 20 mg, and 10 mg in the 100 mg kit product as the drug container containing the drug.
Then, the remaining 20 mg vials were classified as non-selected drug containers, and the selected vials and the container containing the diluted/dissolved solution were delivered to the hospital medicine part.

### Example 11 (recovery of surplus drug)

In Example 10, the pharmacist at the internal pharmacy recovered the unselected 20 mg vial from the kit product delivered from the pharmaceutical wholesaler and stored it in the refrigerated storage in the pharmacy.

### Example 12 (reuse of surplus drug)

Doctor A examined patient D and created a prescription to administer 110 mg of drug C requiring dose adjustment.
For Drug C, a combined 100 mg kit product of 50 mg, 20 mg, 20 mg and 10 mg vials is sold. Then, the hospital medicine part where the doctor works ordered the kit product from the pharmacy through the internal pharmacy.
The internal pharmacy notified the hospital medicine part and the pharmaceutical wholesaler that there was a storage record of the non-selected 20 mg vial generated in Example 11.
It ordered a new 100 mg kit product in a combination of 50 mg, 20 mg, 20 mg and 10 mg vials for the formulation generated in this case.
It classified 50 mg, 20 mg and 20 mg vials as drug containers containing drug and 10 mg vials as non-selected drug containers from the ordered kit product.
It further selected the 20 mg vial stored in Example 7 as the drug container containing the drug and prepared the 110 mg drug required for administration.
The non-selected 10 mg vial was stored in a refrigerated storage.

### Brief Explanation of Drawings

FIG. 1 shows a packaging container according to Example 1.
FIG. 2 shows a packaging container containing a drug according to Example 2.
FIG. 3 shows a kit product comprising a container containing a diluted/dissolved solution containing an infusion solution or pure water according to Example 3, and a plurality of drug enclosing portions having a sealing part, and a series of drug containers containing the drug.
FIG. 4 shows an administration route connected to the kit product according to Example 3.
FIG. 5 is a block diagram showing a system of a computer that executes the method for managing a kit product according to the present invention.
FIG. 6 is a schematic block diagram showing a computer system of a pharmaceutical wholesaler (pharmaceutical company).
FIG. 7 is a schematic block diagram showing a computer system of a dispensing pharmacy (internal pharmacy).
FIG. 8 is a schematic block diagram showing a computer system of a medical institution.
FIG. 9 shows a block diagram showing a distribution form of a drug.
FIG. 10 shows a process diagram for producing the kit product according to the present invention.
FIG. 11 shows a process diagram for producing the kit product according to the present invention.

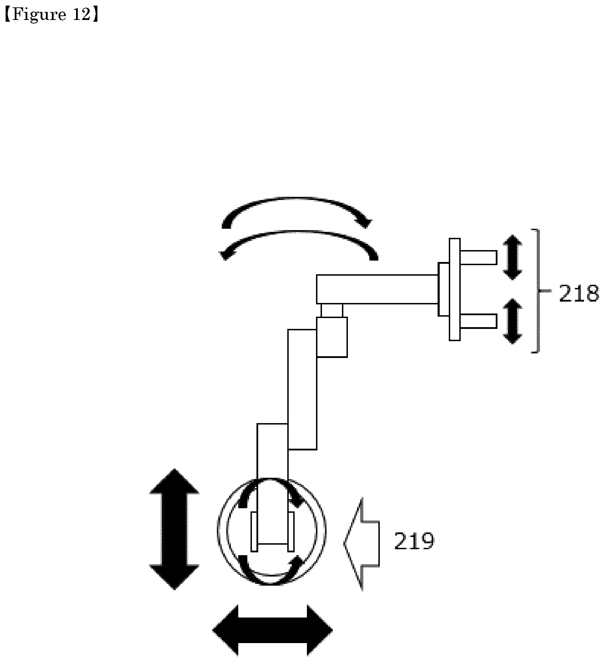

FIG. 12 shows a plan view of a multi-axis robot arm used in the process diagram for producing the kit product according to the present invention.

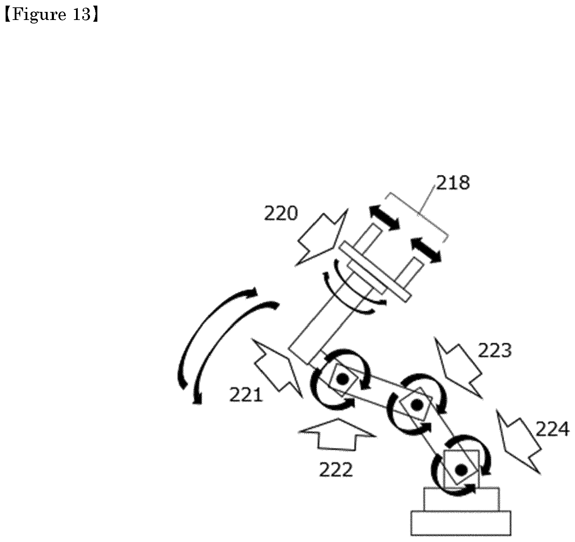

FIG. 13 shows a side view of a multi-axis robot arm used in the process diagram for producing the kit product according to the present invention.

### Description of Reference Numerals

1-1: The first vial connected to the connection port, which comprises the drug-enclosing portion (2-1), the septum (4-1) and the drug (3-1).
1-2: The second vial connected to the connection port, which is composed of the drug-enclosing portion (2-2), the septum (4-2), and the drug (3-2).
1-n: The n-th vial connected to the connection port, which is composed of a drug-enclosing portion (2-n), a septum (4-n), and a drug (3-n).
2-1, 2-2, 2-n: Drug-enclosing portion.
3-1, 3-2, 3-n: Drugs enclosed in vials (1-1, 1-2, 1-n), solid (mainly in the case of freeze-dried preparations), liquid (general Liquid formulation), gas (cyclophosphamide, ifosfamide, bendamustine is said to partially vaporize) in some cases.
4-1, 4-2, 4-n: A septum part forming a part of the vial (1-1, 1-2, 1-n), and a double-ended needle (5-1, 5-2, 5-3) is stuck, and the drug-enclosing portion (2-1, 2-2, 2-n) communicates with the inside of the infusion solution bag part (13), and the drug (3-1, 3-2, 3-n) and the infusion solution (9) can be mixed.
5-1, 5-2, 5-n: Pierce into septum (4-1, 4-2, 4-n) of vials (1-1, 1-2, 1-n) and septum (7-1, 7-2, 7-n) of connection port (8-1, 8-2, 8-n).
   Then, the drug-enclosing portion (2-1, 2-2, 2-n) and the inside of the infusion solution bag part (13) are made to communicate with each other, and the medicine (3-1, 3-2, 3-n) and the infusion solution (13) are connected. A double-ended needle that allows both to mix.
6-1, 6-2, 6-n: A hole formed in the double-ended needle that serves as a flow path for the drug (3-1, 3-2, 3-n) and the infusion solution (13).
7-1, 7-2, 7-n: Septum of connection ports (8-1, 8-2, 8-n), one of the double-ended needles (5-1, 5-2, 5-n) is stuck.
8-1, 8-2, 8-n: The first connection port consists of the double-ended needle (5-1) and the support (18-1) wherein the double-ended needle (5-1) is kept perpendicular to the septum (4-1) of the vial (1-1) and the septum (7-1) of the connecting port (8-1) .
8-2: Second connection port. Consists of a double-ended needle (5-2) and a support (18-2) for keeping the double-ended needle (5-2) perpendicular to the septum (4-2) of the vial (1-2) and the septum (7-2) of the connection port (8-2).
8-n: The n-th connection port. Consists of a double-ended needle (5-n) and a support (18-n) for holding the double-ended needle (5-n) in a position perpendicular to the septum (4-n) of the vial (1-n) and the septum (7-n) of the connection port (8-n).
9: Diluted/dissolved solution containing infusion solution or pure water in the infusion solution bag part (13)
10: A connection part for connecting the infusion solution bag part (13) and the administration route (17).
   Then, it has a septum (19) to which the needle (14) of the administration route is stuck and a female screw structure (11) for fixing the needle (14) so as not to come off.
11: Female screw structure for fixing the administration route (17) to the infusion solution bag part (13).
12: indicates a connection port section.
   To mix the drug inside the vial (1-1, 1-2, 1-n) with the infusion solution (9) in the infusion solution bag part (13), use the vial (1-1, 1-2, 1-n). It must be connected to the infusion solution bag part (13).
   The connection location is called a connection port, and in FIG. 1, it is shown that there are n connection ports (8-1, 8-2, 8-n).
   That is, the first connection port (8-1), the second connection port (8-2), and the nth connection port (8-n).
13: Shows an infusion solution bag part.
   An infusion solution (9) is enclosed inside. This infusion solution serves as a dissolved solution and a diluted solution.
   After the vials (1-1, 1-2, 1-n) are connected to the connection ports, the infusion solution (9) flows into the vials (1-1, 1-2, 1-n), and the dose is adjusted by mixing the infusion solution and drug.
14: A needle of the administration route (17) that pierces the infusion solution bag part (13)
15: Male screw structure for fixing the administration route (17) to the infusion solution bag part (13).
16: Route of administration (17) and needle for puncturing blood vessel of patient.
17: Shows the administration route. It consists of four components, that is, 1. needle (14) for piercing the septum (19), 2. male screw structure (15) for fixing administration route, 3. a needle (16) for piercing the patient, 4. body tube structure (20).
18-1, 18-2, 18-n: Support for keeping the double-ended needles (5-1, 5-2, 5-n) in a position perpendicular to the septum (7-1, 7-2, 7-n) .
19: A septum part to which the needle (14) of the administration route (17) sticks.
   As a result, the infusion solution bag part (9) and the medicines (3-1, 3-2, 3-n) inside the infusion solution bag part (13) flow into the administration route (17).
20: It has a tube structure which is a part of the administration route (17).
100: Computer system of pharmaceutical company (pharmaceutical factory)
100*: Computer of pharmaceutical company (pharmaceutical factory)
110: Computer system for pharmaceutical wholesaler
110*: Pharmaceutical wholesaler computer
120: Computer system of dispensing pharmacy (gate front pharmacy)
121: Computer
122: Output device
123: Input device
124: Reader
125: Storage device
126: Modem
130: Computer system of medical institution
131: Computer
132: receipt computer
133: Reader
134: Modem
140: Computer system of a large medical institution
141: Computer
142: Electronic medical record system
143: Receipt computer
144-1: Terminal of doctors
144-n: Terminal of doctors
145: Modem
150: Internet
160: Output device
161: Pharmaceutical company
162: pharmaceutical wholesaler
163: Hospital
164: Pharmacy
165: Consumer (patient)
166: Management device
167: Management device
168: Management device
169: Management device
170: Customer-specific use data storage device
180: Customer-specific inventory data storage device
190: WWW server
201: Multi-axis robot arm 201
202: Infusion bag with connection port
203: Belt conveyor
204: Container for packaging kit product
204-1: Packaging container for kit product
204-2: Packaging container for kit product
204-11: Packaging container for kit product
205: Belt conveyor
206: Drug vial
207: Belt conveyor
208: drug vial
209: Belt conveyor
210: Drug vial
211: Drug vial
212: Drug vial
213: Drug vial
214: Drug vial
215: Drug vial
216: Drug vial
217: Drug vial
218: Grasping part
219: Joint of robot arm
220: Joint of robot arm
221: Joint of robot arm
222: Joint of robot arm
223: Joint of robot arm
224: Joint of robot arm
231: Multi-axis robot arm 201
241: Multi-axis robot arm 201

### Industrial Applicability

The present invention can provide the medical field with a drug container, a kit product, and a method for managing the kit product, which are useful for preparation suitable for administration of a drug requiring dose adjustment.

## Claims

1. A packaging container for a drug having a recording medium and/or an identification mark for recording and updating one or more arbitrary information(s) selected from the group consisting of the information (1) to (9):
(1) An active ingredient of a drug
(2) Usage of a drug
(3) Drug dose
(4) Date of manufacture of a drug
(5) Expiration date of a drug
(6) Drug shipping date of a drug
(7) Traceability of drug delivery route from manufacturing to usage of a drug, and storage conditions (eg. temperature, light (dark/light), humidity, etc.) including storage temperature and changes thereof
(8) Information including the administration date of a drug and a breakdown of the administration
(9) Amount of drug presented in drug-enclosing portion

2. The drug container according to claim 1, further containing the drug, which corresponds to (1) to (9) information about the drug concerned.

3. The drug container according to claim 2, wherein the drug dose is fixed or determined based on patient information of the usage and dose for the patient.

4. The drug container according to claim 2 or 3, wherein the drug for a patient is an injection for a disease selected from the group consisting of 1) to 17) below:
1) Sensory organ agents
2) Central nervous system agents
3) Peripheral nerve agents
4) Cardiovascular agents
5) Respiratory agents
6) Gastrointestinal Drug
7) Hormonal agents (antihormonal agents)
8) Drugs for blood and body fluids
9) Other metabolic drugs
10) Tumor drugs
11) Radiopharmaceuticals
12) Antibiotic agents
13) Chemotherapeutic agents
14) Biological agents
15) Parasitic drugs
16) Diagnostic drugs (excluding in-vitro diagnostic drugs)
17) Non-alkaloid narcotics.

5. A series of drug containers according to claim 2, each of which contains at least one drug dose selected from the group consisting of 1x, 2x, 3x, 4x, 5x, 6x, 7x, 8x, 9x, 10x, 20x, 30x, 40x, 50x, 60x, 70x, 80x, 90x and 100x (wherein x indicates a multiplier of 0.001, 0.01, 0.1, 1.0, 10, 100, and 1000 depending on the approved prescribed dose of a drug (unit: µg, mg, g) to be used for dose adjustment of the administration drug.

6. A kit product comprising a container containing a diluted or dissolved solution including an infusion solution or pure water and a plurality of drug enclosing portions having sealing part, wherein said drug is enclosed in the packaging container according to claim 1, and the container and the drug enclosing portions are brought into communication with each other by connecting the drug container in which the drug is enclosed to the packaging container via the sealing part and opening the sealing part, and the series of drug containers having the drug according to claim 5.

7. The kit product according to claim 6 wherein the drug can be prepared by being placed in a container containing a diluted or dissolved solution, and the dose of the drug is selected based on patient information, by selecting the drug and the drug enclosing portions to adjust the dose of the drug and the dosage of the drug based on significant digit of the dose **characterized in** a constitution of a drug to be administered and a drug to be separated and not administered.

8. The kit product according to claim 6 or 7, which comprises a container containing a diluted or dissolved solution including an infusion solution or pure water, plural drug enclosing portions having sealing part(s), and plural drug containers selected from a series of drug containers.

9. A method for managing a kit product containing a selected drug container and a non-selected drug container **characterized in** comprising the following steps (1) to (7):
(1) A step of providing medication prescription
(2) A step of providing the kit product according to claim 6 based on the medication prescription
(3) A step of providing a drug container selected for dispensing of the kit product
(4) A step of drug management including a step of providing a non-selected drug container for dispensing the kit product (5) Classification of selected drug containers and non-selected drug containers in the above kit product
(6) Providing data on the selected drug containers and information thereof based on the above classification results
(7) Providing data on non-selected drug containers and information thereof based on the above classification results.

10. A control method of computer wherein the computer system includes a data storage area which is connected to the terminals of the medical provider, the dispensing provider, and the pharmaceutical provider and a data storage unit where the drug manufacturer and/or drug supplier store the addresses of the terminals thereof, and the data storage area is executed to manage a selected drug container and the drug thereof and a non-selected drug container and the drug thereof **characterized in** including the following steps (1) to (8) via computer network:
(1) A step of receiving data of medication prescription from the terminal of the medical provider
(2) A step of receiving the data of the kit product according to claim 6 from the terminals of the drug manufacturer and/or the drug supplier based on the data of the medication prescription
(3) A step of receiving the data of a selected drug container for dispensing of the kit product at terminals of a dispensing provider, a pharmaceutical provider, and a drug manufacturer and/or a drug supplier
(4) A step of controlling a computer including a step of receiving the data of a non-selected drug container for dispensing of the kit product at terminals of the dispensing provider, the pharmaceutical provider, and the drug manufacturer and/or the drug supplier
(5) Classification of selected drug containers and non-selected drug containers in the above kit product
(6) Accepting the data of the selected drug container and information thereof based on the above classification result at the terminals of the dispensing provider, the pharmaceutical provider, and the drug manufacturer and/or drug supplier
(7) Separation and collection of the non-selected drug containers on the basis of the above classification result, in response to receiving the data regarding the non-selected drug containers and information thereof at the terminals of the dispensing provider, the pharmaceutical provider, and the drug manufacturer and/or drug supplier
(8) The step of transmitting the processing information regarding the separation and the collection to the terminals of the medical provider, the dispensing provider, and the pharmaceutical provider.

11. A computer system wherein the computer system includes a data storage area which is connected to the terminals of the medical provider, the dispensing provider and a data storage unit where the drug manufacturer and/or drug supplier store the addresses of the terminals thereof, and the data storage area carries out management of a selected drug container and the drug thereof and a non-selected drug container and the drug thereof **characterized in** including the following steps (1) to (8) via computer network:
(1) A step of receiving data of medication prescription from the terminal of the medical provider
(2) A step of receiving the data of the kit product according to claim 6 from the terminals of the drug manufacturer and/or the drug supplier based on the data of the medication prescription
(3) A step of receiving the data of a selected drug container for dispensing of the kit product at terminals of a dispensing provider, a pharmaceutical provider, and a drug manufacturer and/or a drug supplier
(4) A step of controlling a computer including a step of receiving the data of a non-selected drug container for dispensing of the kit product at terminals of the dispensing provider, the pharmaceutical provider, and the drug manufacturer and/or the drug supplier
(5) Classification of selected drug containers and non-selected drug containers in the above kit product
(6) Accepting the data of the selected drug container and information thereof based on the above classification result at the terminals of the dispensing provider, the pharmaceutical provider, and the drug manufacturer and/or drug supplier
(7) Separation and collection of the non-selected drug containers on the basis of the above classification result, in response to receiving the data regarding the non-selected drug containers and information thereof at the terminals of the dispensing provider, the pharmaceutical provider, and the drug manufacturer and/or the drug supplier
(8) Transmission of the processing information regarding the separation and the collection to the terminals of the medical provider, the dispensing provider, and the pharmaceutical provider.

12. A computer program wherein the computer system includes a data storage area which is connected to the terminals of the medical provider, the dispensing provider, and the pharmaceutical provider and a data storage unit where the drug manufacturer and/or drug supplier store the addresses of the terminals thereof, and the data storage area carries out management of a selected drug container and the drug thereof and a non-selected drug container and the drug thereof **characterized in** performing the following steps (1) to (8) via computer network:
(1) A step of receiving data of medication prescription from the terminal of the medical provider
(2) A step of receiving the data of the kit product according to claim 6 from the terminals of the drug manufacturer and/or the drug supplier based on the data of the medication prescription
(3) A step of receiving the data of a selected drug container for dispensing of the kit product at terminals of a dispensing provider, a pharmaceutical provider, and a drug manufacturer and/or a drug supplier
(4) A step of controlling a computer including a step of receiving the data of a non-selected drug container for dispensing of the kit product at terminals of the dispensing provider, the pharmaceutical provider, and the drug manufacturer and/or the drug supplier
(5) Classification of selected drug containers and non-selected drug containers in the above kit product
(6) Accepting the data of the selected drug container and information thereof based on the above classification result at the terminals of the dispensing provider, the pharmaceutical provider, and the drug manufacturer and/or drug supplier
(7) Separation and collection of the non-selected drug containers on the basis of the above classification result, in response to receiving the data regarding the non-selected drug containers and information thereof at the terminals of the dispensing provider, the pharmaceutical provider, and the drug manufacturer and/or the drug supplier based on the classification result
(8) Transmitting the processing information regarding the separation and the collection to the terminals of the medical provider, the dispensing provider, and the pharmaceutical provider.

13. A method for producing a kit product **characterized in** comprising preparation of a series of drug containers according to claim 2, and combination of plural drug-enclosing portions having a container containing the diluted or dissolved solution including an infusion solution or pure water according to claim 6 and a sealing part, and the series of drug containers by using the method for managing a kit product according to claims 9 to 11.

14. A process for producing a kit product **characterized in** comprising preparation of a series of drug containers according to claim 2, and combination plural drug-enclosing portions having a container containing the diluted or dissolved solution including an infusion solution or pure water according to claim 6 and a sealing part, and the series of drug containers by using the method for managing a kit product according to claims 9 to 11.

15. The method for producing a kit product according to claim 13 **characterized in** further using the recovered non-selected drug container according to (4) of claim 9.

16. A method for separating, recovering and/or reusing a non-selected drug container, **characterized in** combining the method for managing a kit product according to claim 9 and the method for preparation of a kit product according to claim 13.

17. A control method of computer for administration of the kit product according to claim 10 wherein the computer system includes a data storage area which is connected to each terminal of medical workers and a data storage unit where the medical workers store each the addresses of the terminals thereof, the data storage area is **characterized in** including the following means (1) to (3) via computer network:
(1) Means for a doctor to transmit a medication prescription relating to the kit product according to claim 6, and to transmit and receive the data of the medication prescription among the terminals of medical workers other than the doctor
(2)Means for transmitting or receiving the data regarding the selected drug containers and the information thereof and the data regarding the non-selected drug containers and the information thereof, and/or the data on a container containing a diluted or dissolved solution including an infusion solution or pure water and plural drug-enclosing portions having sealing part(s) among each terminal of medical workers in case of the kit product according to claim 6 based on the data of the medication prescription, classifying selected drug containers and non-selected drug containers is settled
(3) Means for transmitting and receiving processing information regarding separation and collection of the non-selected drug container among each terminal of medical workers.

18. A computer system for administration of the kit product according to claim 10, which includes a data storage area which is connected to each terminal of medical workers and a data storage unit where the medical workers store each the addresses of the terminals thereof, the data storage area is **characterized in** including the following means (1) to (3) via computer network:
(1) Means for a doctor to transmit a medication prescription relating to the kit product according to claim 6, and to transmit and receive the data of the medication prescription among the terminals of medical workers other than the doctor
(2) Means for transmitting or receiving the data regarding the selected drug containers and the information thereof and the data regarding the non-selected drug containers and the information thereof, and/or the data on a container containing a diluted or dissolved solution including an infusion solution or pure water and plural drug-enclosing portions having sealing part(s) among each terminal of medical workers in case of the kit product according to claim 6 based on the data of the medication prescription, classifying selected drug containers and non-selected drug containers is settled
(3) Means for transmitting and receiving processing information regarding separation and collection of the non-selected drug container among each terminal of medical workers.

19. A computer program for administration of the kit product according to claim 10 wherein the computer system includes a data storage area which is connected to each terminal of medical workers and a data storage unit where the medical workers store each the addresses of the terminals thereof, the data storage area is **characterized in** including the following means (1) to (3) via computer network:
(1) Means for a doctor to transmit a medication prescription relating to the kit product according to claim 6, and to transmit and receive the data of the medication prescription among the terminals of medical workers other than the doctor.
(2) Means for transmitting or receiving the data regarding the selected drug containers and the information thereof and the data regarding the non-selected drug containers and the information thereof, and/or the data on a container containing a diluted or dissolved solution including an infusion solution or pure water and plural drug-enclosing portions having sealing part(s) among each terminal of medical workers in case of the kit product according to claim 6 based on the data of the medication prescription, classifying selected drug containers and non-selected drug containers is settled
(3) Means for transmitting and receiving processing information regarding separation and collection of the non-selected drug container among each terminal of medical workers.
